# EUROPEAN PATENT APPLICATION

(11) **EP 4 382 538 A1**
(43) Date of publication of application: **12.06.2024**
(21) Application number: 22852051.6
(22) Date of filing: 29.07.2022
(51) Int. Cl.: C07K 16/28, A61K 47/68

(54) **ANTI-CD79B×CD3 BISPECIFIC ANTIBODY AND USE THEREOF**

(30) Priority: 02.08.2021 CN 202110881474
(71) Applicant: Innovent Biologics (Suzhou) Co., Ltd., Suzhou, Jiangsu 215123 (CN)
(72) Inventor: WU, Weiwei, Suzhou, Jiangsu 215123 (CN); WANG, Jie, Suzhou, Jiangsu 215123 (CN); HE, Kaijie, Suzhou, Jiangsu 215123 (CN); LI, Li, Suzhou, Jiangsu 215123 (CN); ZHOU, Shuaixiang, Suzhou, Jiangsu 215123 (CN)
(74) Representative: Sagittarius IP
(86) International application number: PCT/CN2022/108881
(87) International publication number: WO 2023/011338

(57) **Abstract**

The present invention relates to an antibody specifically binding to CD79b and a bispecific antibody specifically binding to CD79b and CD3, nucleic acids encoding the anti-CD79b antibody and the anti-CD79b×CD3 bispecific antibody, vectors comprising the nucleic acids, host cells comprising the nucleic acids or the vectors, and pharmaceutical compositions comprising the antibodies or antigen-binding fragments thereof.

## Description

### TECHNICAL FIELD

The present invention generally relates to the field of immunology and antibody engineering. In particular, the present invention relates to a CD79b monospecific antibody and a novel bispecific antibody specifically binding to CD79b and CD3. Furthermore, the present invention relates to nucleic acids encoding the antibodies, vectors comprising the nucleic acids, host cells comprising the nucleic acids or the vectors, and pharmaceutical compositions comprising the antibodies or antigen-binding fragments thereof. In addition, the present invention relates to use of these antibodies, pharmaceutical compositions, and the like in the immunotherapy, prevention, and/or diagnosis of diseases.

### BACKGROUND

CD79b belongs to the immunoglobulin superfamily and forms a heterodimer with CD79a, and the two and the surface globulin constitute a BCR receptor complex. BCR itself has no signaling domain, and once an antigen binds to BCR, the CD79a/b heterodimer is responsible for signaling downstream, and this is critical for maintaining overall B cell function. Research shows that after the CD79b is knocked out, B cells are limited to the pre-B stage and cannot develop and mature further.

B-cell non-Hodgkin's lymphoma makes up about 85% of NHL cases. The recurrence rate is high following the first-line treatment of B-cell NHL(the recurrence rate is 30-40% following the first-line treatment of DLBCL), and the relapsed patients are faced with poor prognosis (the OS of DLBCL is 9-12 months on average) and limited late-stage therapy. Meanwhile, the number of new cases and recurrent and refractory cases is huge every year (about 80,000 new cases every year in both China and the United States), and particularly in China, the number of deaths per year reaches about 50,000, which is more than 2 times that in the United States. Antibody-drug conjugates against CD79b have shown certain clinical efficacy in NHL patients, but some patients have developed resistance to them, and there is still a great unmet need of patients for improved therapies against NHL.

In recent years, bispecific antibody-based immunotherapies have developed rapidly; they are capable of simultaneously binding to cytotoxic cells and surface antigens on tumor cells, thus mediating the killing of tumor cells by cytotoxic cells. Compared with antibody-drug conjugates, they have certain advantages in efficacy and safety.

The present invention meets this need by providing a bispecific antibody that binds to CD79b and CD3 with high target specificity and high affinity, in particular a bispecific antibody that allows T cells to be recruited to the periphery of tumor cells by binding to CD79b expressed on the surface of the tumor cells.

### SUMMARY

In one aspect, the present invention relates to a novel antibody or antigen-binding fragment thereof that binds to CD79b.

In some embodiments, the present invention provides an antibody or an antigen-binding fragment thereof that binds to CD79b, which comprises a heavy chain variable region VH and/or a light chain variable region VL, wherein
1) the VH comprises three complementarity determining regions (CDRs) HCDR1, HCDR2, and HCDR3 contained in a VH set forth in SEQ ID NO: 4, and the VL comprises LCDR1, LCDR2, and LCDR3 contained in a VL set forth in SEQ ID NO: 9;
2) the VH comprises three complementarity determining regions (CDRs) HCDR1, HCDR2, and HCDR3 contained in a VH set forth in SEQ ID NO: 14, and the VL comprises LCDR1, LCDR2, and LCDR3 contained in a VL set forth in SEQ ID NO: 19.

In some embodiments, the present invention provides an antibody or an antigen-binding fragment thereof that binds to CD79b, which comprises a heavy chain variable region VH and/or a light chain variable region VL, wherein
(i) the VH comprises complementarity determining regions (CDRs) HCDR1, HCDR2, and HCDR3, wherein the HCDR1 comprises an amino acid sequence set forth in SEQ ID NO: 1 or SEQ ID NO: 11; the HCDR2 comprises an amino acid sequence set forth in SEQ ID NO: 2 or SEQ ID NO: 12; the HCDR3 comprises an amino acid sequence set forth in SEQ ID NO: 3 or SEQ ID NO: 13;
   and/or
(ii) the VL comprises complementarity determining regions (CDRs) LCDR1, LCDR2, and LCDR3, wherein the LCDR1 comprises an amino acid sequence set forth in SEQ ID NO: 6 or SEQ ID NO: 16; the LCDR2 comprises an amino acid sequence set forth in SEQ ID NO: 7 or SEQ ID NO: 17; the LCDR3 comprises an amino acid sequence set forth in SEQ ID NO: 8 or SEQ ID NO: 18.

In some embodiments, the present invention provides an antibody or an antigen-binding fragment thereof that binds to CD79b, which comprises a heavy chain variable region VH and/or a light chain variable region VL, wherein
1) the VH comprises complementarity determining regions (CDRs) HCDR1, HCDR2, and HCDR3, wherein the HCDR1 comprises the amino acid sequence set forth in SEQ ID NO: 1, the HCDR2 comprises the amino acid sequence set forth in SEQ ID NO: 2, and the HCDR3 comprises the amino acid sequence set forth in SEQ ID NO: 3; the VL comprises complementarity determining regions (CDRs) LCDR1, LCDR2, and LCDR3, wherein the LCDR1 comprises the amino acid sequence set forth in SEQ ID NO: 6, the LCDR2 comprises the amino acid sequence set forth in SEQ ID NO: 7, and the LCDR3 comprises the amino acid sequence set forth in SEQ ID NO: 8;
2) the VH comprises complementarity determining regions (CDRs) HCDR1, HCDR2, and HCDR3, wherein the HCDR1 comprises the amino acid sequence set forth in SEQ ID NO: 11, the HCDR2 comprises the amino acid sequence set forth in SEQ ID NO: 12, and the HCDR3 comprises the amino acid sequence set forth in SEQ ID NO: 13; the VL comprises complementarity determining regions (CDRs) LCDR1, LCDR2, and LCDR3, wherein the LCDR1 comprises the amino acid sequence set forth in SEQ ID NO: 16, the LCDR2 comprises the amino acid sequence set forth in SEQ ID NO: 17, and the LCDR3 comprises the amino acid sequence set forth in SEQ ID NO: 18.

In some embodiments, the present invention provides an antibody or an antigen-binding fragment thereof that binds to CD79b, which comprises a heavy chain variable region VH and/or a light chain variable region VL, wherein
(a) the heavy chain variable region VH
   (i) comprises or consists of an amino acid sequence having at least 90%, 91%, 92%, 93%, 94%, 95%, 96%, 97%, 98%, or 99% identity to the amino acid sequence set forth in SEQ ID NO: 4 or SEQ ID NO: 14; or
   (ii) comprises or consists of the amino acid sequence set forth in SEQ ID NO: 4 or SEQ ID NO: 14; or
   (iii) comprises an amino acid sequence having one or more (preferably no more than 10, and more preferably no more than 5, 4, 3, 2, or 1) amino acid changes (preferably amino acid replacements, and more preferably conservative amino acid replacements) compared with the amino acid sequence set forth in SEQ ID NO: 4 or SEQ ID NO: 14, wherein preferably, the amino acid changes do not occur in the CDRs;
      and/or
(b) the light chain variable region VL
   (i) comprises or consists of an amino acid sequence having at least 90%, 91%, 92%, 93%, 94%, 95%, 96%, 97%, 98%, or 99% identity to the amino acid sequence set forth in SEQ ID NO: 9 or SEQ ID NO: 19;
   (ii) comprises or consists of the amino acid sequence set forth in SEQ ID NO: 9 or SEQ ID NO: 19; or
   (iii) comprises an amino acid sequence having one or more (preferably no more than 10, and more preferably no more than 5, 4, 3, 2, or 1) amino acid changes (preferably amino acid replacements, and more preferably conservative amino acid replacements) compared with the amino acid sequence set forth in SEQ ID NO: 9 or SEQ ID NO: 19, wherein preferably, the amino acid changes do not occur in the CDRs.

In some embodiments, the present invention provides an antibody or an antigen-binding fragment thereof that binds to CD79b, which comprises:
1) a heavy chain variable region VH comprising an amino acid sequence having at least 90%, 91%, 92%, 93%, 94%, 95%, 96%, 97%, 98%, or 99% identity to the amino acid sequence set forth in SEQ ID NO: 4, and a light chain variable region VL comprising an amino acid sequence having at least 90%, 91%, 92%, 93%, 94%, 95%, 96%, 97%, 98%, or 99% identity to the amino acid sequence set forth in SEQ ID NO: 9;
2) a heavy chain variable region VH comprising an amino acid sequence having at least 90%, 91%, 92%, 93%, 94%, 95%, 96%, 97%, 98%, or 99% identity to the amino acid sequence set forth in SEQ ID NO: 14, and a light chain variable region VL comprising an amino acid sequence having at least 90%, 91%, 92%, 93%, 94%, 95%, 96%, 97%, 98%, or 99% identity to the amino acid sequence set forth in SEQ ID NO: 19.

In some embodiments, the present invention provides an antibody or an antigen-binding fragment thereof that binds to CD79b, which comprises:
1) a heavy chain variable region VH comprising the amino acid sequence set forth in SEQ ID NO: 4 and a light chain variable region VL comprising the amino acid sequence set forth in SEQ ID NO: 14;
2) a heavy chain variable region VH comprising the amino acid sequence set forth in SEQ ID NO: 9 and a light chain variable region VL comprising the amino acid sequence set forth in SEQ ID NO: 19.

In some embodiments, the present invention provides an antibody or an antigen-binding fragment thereof that binds to CD79b, which comprises a heavy chain and/or a light chain, wherein
(a) the heavy chain
   (i) comprises or consists of an amino acid sequence having at least 85%, 90%, 91%, 92%, 93%, 94%, 95%, 96%, 97%, 98%, or 99% identity to an amino acid sequence set forth in SEQ ID NO: 5 or SEQ ID NO: 15;
   (ii) comprises or consists of the amino acid sequence set forth in SEQ ID NO: 5 or SEQ ID NO: 15; or
   (iii) comprises an amino acid sequence having one or more (preferably no more than 20 or 10, and more preferably no more than 5, 4, 3, 2, or 1) amino acid changes (preferably amino acid replacements, and more preferably conservative amino acid replacements) compared with the amino acid sequence set forth in SEQ ID NO: 5 or SEQ ID NO: 15, wherein preferably, the amino acid changes do not occur in the CDRs of the heavy chain, and more preferably, the amino acid changes do not occur in the heavy chain variable region;
      and/or
(b) the light chain
   (i) comprises or consists of an amino acid sequence having at least 85%, 90%, 91%, 92%, 93%, 94%, 95%, 96%, 97%, 98%, or 99% identity to an amino acid sequence set forth in SEQ ID NO: 10 or SEQ ID NO: 20;
   (ii) comprises or consists of the amino acid sequence set forth in SEQ ID NO: 10 or SEQ ID NO: 20; or
   (iii) comprises an amino acid sequence having one or more (preferably no more than 20 or 10, and more preferably no more than 5, 4, 3, 2, or 1) amino acid changes (preferably amino acid replacements, and more preferably conservative amino acid replacements) compared with the amino acid sequence set forth in SEQ ID NO: 10 or SEQ ID NO: 20, wherein preferably, the amino acid changes do not occur in the CDRs of the light chain, and more preferably, the amino acid changes do not occur in the light chain variable region.

In some embodiments, the present invention provides an antibody or an antigen-binding fragment thereof that binds to CD79b, which comprises:
1) a heavy chain comprising an amino acid sequence having at least 85%, 90%, 91%, 92%, 93%, 94%, 95%, 96%, 97%, 98%, or 99% identity to the amino acid sequence set forth in SEQ ID NO: 5, and a light chain comprising an amino acid sequence having at least 85%, 90%, 91%, 92%, 93%, 94%, 95%, 96%, 97%, 98%, or 99% identity to the amino acid sequence set forth in SEQ ID NO: 10;
2) a heavy chain comprising an amino acid sequence having at least 85%, 90%, 91%, 92%, 93%, 94%, 95%, 96%, 97%, 98%, or 99% identity to the amino acid sequence set forth in SEQ ID NO: 15, and a light chain comprising an amino acid sequence having at least 85%, 90%, 91%, 92%, 93%, 94%, 95%, 96%, 97%, 98%, or 99% identity to the amino acid sequence set forth in SEQ ID NO: 20.

In some embodiments, the present invention provides an antibody or an antigen-binding fragment thereof that binds to CD79b, which comprises:
1) a heavy chain comprising the amino acid sequence set forth in SEQ ID NO: 5 and a light chain comprising the amino acid sequence set forth in SEQ ID NO: 10;
2) a heavy chain comprising the amino acid sequence set forth in SEQ ID NO: 15 and a light chain comprising the amino acid sequence set forth in SEQ ID NO: 20.

In some embodiments, the present invention provides an isolated nucleic acid encoding the antibody or the antigen-binding fragment thereof that binds to CD79b of the present invention, a vector comprising the nucleic acid, and a host cell comprising the nucleic acid or the vector.

In some embodiments, the present invention provides a method for preparing the antibody or the antigen-binding fragment thereof that binds to CD79b of the present invention, wherein the method comprises culturing the host cell described herein under conditions suitable for expressing the nucleic acid described herein.

In some embodiments, the present invention provides an immunoconjugate and a pharmaceutical composition comprising the antibody or the antigen-binding fragment thereof that binds to CD79b of the present invention.

In some embodiments, the present invention also provides use of the antibody or the antigen-binding fragment thereof that binds to CD79b, the immunoconjugate, or the pharmaceutical composition of the present invention in preparing a medicament for the prevention and/or treatment of a tumor.

In some embodiments, the present invention also provides a method for preventing and/or treating a tumor, which comprises administering to a subject an effective amount of the antibody or the antigen-binding fragment thereof that binds to CD79b, the immunoconjugate, or the pharmaceutical composition of the present invention.

The present invention also relates to a method for detecting CD79b in a sample, which comprises: (a) contacting the antibody or the antigen-binding fragment thereof described herein; and (b) detecting a complex formed by the antibody or the antigen-binding fragment thereof and CD79b.

In another aspect, the present invention also discloses a novel bispecific antibody targeting both CD79b and CD3, a polynucleotide encoding the bispecific antibody, a vector comprising the polynucleotide, a host cell comprising the polynucleotide or the vector, and use of the bispecific antibody in the treatment, prevention, and/or diagnosis of a disease related to CD79b activity in an individual.

Accordingly, in one aspect, the present invention provides a bispecific antibody that specifically binds to both CD79b and CD3 (anti-CD79b×CD3 bispecific antibody), which comprises (i) an anti-CD79b antibody or a fragment thereof and (ii) an anti-CD3 antibody or a fragment thereof. In one embodiment, the present invention provides an anti-CD79b×CD3 bispecific antibody in a 1+1 format consisting of four polypeptide chains that are bilaterally symmetric, wherein the left half consists of a first light chain and a first heavy chain, and the right half consists of a second heavy chain and a second light chain; the first light chain and the first heavy chain are the heavy chain and the light chain of an antibody targeting CD79b or CD3, and the second heavy chain and the second light chain are the heavy chain and the light chain of an antibody targeting CD3 or CD79b.

In one embodiment, the present invention provides the anti-CD79b×CD3 bispecific antibody in a 1+1 format, wherein the first light chain and the first heavy chain specifically bind to CD79b, and the second heavy chain and the second light chain specifically bind to CD3. In a specific embodiment, the anti-CD79b×CD3 bispecific antibody provided herein comprises a first light chain, a first heavy chain, a second heavy chain, and a second light chain, wherein the first light chain comprises 3 light chain CDRs contained in SEQ ID NO: 9, 19, or 29, the first heavy chain comprises 3 heavy chain CDRs contained in SEQ ID NO: 4, 14, or 24, the second heavy chain comprises 3 heavy chain CDRs contained in SEQ ID NO: 34 or 44, and the second light chain comprises 3 light chain CDRs contained in SEQ ID NO: 39 or 49.

In a specific embodiment, the anti-CD79b×CD3 bispecific antibody provided herein comprises a first light chain, a first heavy chain, a second heavy chain, and a second light chain, wherein the first light chain and the first heavy chain bind to CD79b, and the second heavy chain and the second light chain bind to CD3, wherein:
1) the first light chain comprises 3 light chain CDRs contained in SEQ ID NO: 9, the first heavy chain comprises 3 heavy chain CDRs contained in SEQ ID NO: 4, the second heavy chain comprises 3 heavy chain CDRs contained in SEQ ID NO: 34, and the second light chain comprises 3 light chain CDRs contained in SEQ ID NO: 39;
2) the first light chain comprises 3 light chain CDRs contained in SEQ ID NO: 9, the first heavy chain comprises 3 heavy chain CDRs contained in SEQ ID NO: 4, the second heavy chain comprises 3 heavy chain CDRs contained in SEQ ID NO: 44, and the second light chain comprises 3 light chain CDRs contained in SEQ ID NO: 49;
3) the first light chain comprises 3 light chain CDRs contained in SEQ ID NO: 19, the first heavy chain comprises 3 heavy chain CDRs contained in SEQ ID NO: 14, the second heavy chain comprises 3 heavy chain CDRs contained in SEQ ID NO: 34, and the second light chain comprises 3 light chain CDRs contained in SEQ ID NO: 39;
4) the first light chain comprises 3 light chain CDRs contained in SEQ ID NO: 19, the first heavy chain comprises 3 heavy chain CDRs contained in SEQ ID NO: 14, the second heavy chain comprises 3 heavy chain CDRs contained in SEQ ID NO: 44, and the second light chain comprises 3 light chain CDRs contained in SEQ ID NO: 49;
5) the first light chain comprises 3 light chain CDRs contained in SEQ ID NO: 29, the first heavy chain comprises 3 heavy chain CDRs contained in SEQ ID NO: 24, the second heavy chain comprises 3 heavy chain CDRs contained in SEQ ID NO: 34, and the second light chain comprises 3 light chain CDRs contained in SEQ ID NO: 39; or
6) the first light chain comprises 3 light chain CDRs contained in SEQ ID NO: 29, the first heavy chain comprises 3 heavy chain CDRs contained in SEQ ID NO: 24, the second heavy chain comprises 3 heavy chain CDRs contained in SEQ ID NO: 44, and the second light chain comprises 3 light chain CDRs contained in SEQ ID NO: 49.

In a specific embodiment, the anti-CD79b×CD3 bispecific antibody provided herein comprises a first light chain, a first heavy chain, a second heavy chain, and a second light chain, wherein the first light chain comprises a VL comprising 3 light chain CDRs contained in SEQ ID NO: 9, 19, or 29 and having at least 90% identity to the SEQ ID NO: 9, 19, or 29, the first heavy chain comprises a VH comprising 3 heavy chain CDRs contained in SEQ ID NO: 4, 14, or 24 and having at least 90% identity to the SEQ ID NO: 4, 14, or 24, the second heavy chain comprises a VH comprising 3 heavy chain CDRs contained in SEQ ID NO: 34 or 44 and having at least 90% identity to the SEQ ID NO: 34 or 44, and the second light chain comprises a VL comprising 3 light chain CDRs contained in SEQ ID NO: 39 or 49 and having at least 90% identity to the SEQ ID NO: 39 or 49.

In a specific embodiment, the anti-CD79b×CD3 bispecific antibody provided herein comprises a first light chain, a first heavy chain, a second heavy chain, and a second light chain, wherein:
1) the first light chain comprises a VL comprising 3 light chain CDRs contained in SEQ ID NO: 9 and having at least 90% identity to the SEQ ID NO: 9, the first heavy chain comprises a VH comprising 3 heavy chain CDRs contained in SEQ ID NO: 4 and having at least 90% identity to the SEQ ID NO: 4, the second heavy chain comprises a VH comprising 3 heavy chain CDRs contained in SEQ ID NO: 34 and having at least 90% identity to the SEQ ID NO: 34, and the second light chain comprises a VL comprising 3 light chain CDRs contained in SEQ ID NO: 39 and having at least 90% identity to the SEQ ID NO: 39;
2) the first light chain comprises a VL comprising 3 light chain CDRs contained in SEQ ID NO: 9 and having at least 90% identity to the SEQ ID NO: 9, the first heavy chain comprises a VH comprising 3 heavy chain CDRs contained in SEQ ID NO: 4 and having at least 90% identity to the SEQ ID NO: 4, the second heavy chain comprises a VH comprising 3 heavy chain CDRs contained in SEQ ID NO: 44 and having at least 90% identity to the SEQ ID NO: 44, and the second light chain comprises a VL comprising 3 light chain CDRs contained in SEQ ID NO: 49 and having at least 90% identity to the SEQ ID NO: 49;
3) the first light chain comprises a VL comprising 3 light chain CDRs contained in SEQ ID NO: 19 and having at least 90% identity to the SEQ ID NO: 19, the first heavy chain comprises a VH comprising 3 heavy chain CDRs contained in SEQ ID NO: 14 and having at least 90% identity to the SEQ ID NO: 14, the second heavy chain comprises a VH comprising 3 heavy chain CDRs contained in SEQ ID NO: 34 and having at least 90% identity to the SEQ ID NO: 34, and the second light chain comprises a VL comprising 3 light chain CDRs contained in SEQ ID NO: 39 and having at least 90% identity to the SEQ ID NO: 39;
4) the first light chain comprises a VL comprising 3 light chain CDRs contained in SEQ ID NO: 19 and having at least 90% identity to the SEQ ID NO: 19, the first heavy chain comprises a VH comprising 3 heavy chain CDRs contained in SEQ ID NO: 14 and having at least 90% identity to the SEQ ID NO: 14, the second heavy chain comprises a VH comprising 3 heavy chain CDRs contained in SEQ ID NO: 44 and having at least 90% identity to the SEQ ID NO: 44, and the second light chain comprises a VL comprising 3 light chain CDRs contained in SEQ ID NO: 49 and having at least 90% identity to the SEQ ID NO: 49;
5) the first light chain comprises a VL comprising 3 light chain CDRs contained in SEQ ID NO: 29 and having at least 90% identity to the SEQ ID NO: 29, the first heavy chain comprises a VH comprising 3 heavy chain CDRs contained in SEQ ID NO: 24 and having at least 90% identity to the SEQ ID NO: 24, the second heavy chain comprises a VH comprising 3 heavy chain CDRs contained in SEQ ID NO: 34 and having at least 90% identity to the SEQ ID NO: 34, and the second light chain comprises a VL comprising 3 light chain CDRs contained in SEQ ID NO: 39 and having at least 90% identity to the SEQ ID NO: 39; or
6) the first light chain comprises a VL comprising 3 light chain CDRs contained in SEQ ID NO: 29 and having at least 90% identity to the SEQ ID NO: 29, the first heavy chain comprises a VH comprising 3 heavy chain CDRs contained in SEQ ID NO: 24 and having at least 90% identity to the SEQ ID NO: 24, the second heavy chain comprises a VH comprising 3 heavy chain CDRs contained in SEQ ID NO: 44 and having at least 90% identity to the SEQ ID NO: 44, and the second light chain comprises a VL comprising 3 light chain CDRs contained in SEQ ID NO: 49 and having at least 90% identity to the SEQ ID NO: 49.

In a specific embodiment, the anti-CD79b×CD3 bispecific antibody provided herein comprises a first light chain, a first heavy chain, a second heavy chain, and a second light chain, wherein:
1) the first light chain comprises LCDR1, LCDR2, and LCDR3, wherein the LCDR1 comprises or consists of an amino acid sequence of SEQ ID NO: 6, the LCDR2 comprises or consists of an amino acid sequence of SEQ ID NO: 7, and the LCDR3 comprises or consists of an amino acid sequence of SEQ ID NO: 8; the first heavy chain comprises HCDR1, HCDR2, and HCDR3, wherein the HCDR1 comprises or consists of an amino acid sequence of SEQ ID NO: 1, the HCDR2 comprises or consists of an amino acid sequence of SEQ ID NO: 2, and the HCDR3 comprises or consists of an amino acid sequence of SEQ ID NO: 3; the second heavy chain comprises HCDR1, HCDR2, and HCDR3, wherein the HCDR1 comprises or consists of an amino acid sequence of SEQ ID NO: 31, the HCDR2 comprises or consists of an amino acid sequence of SEQ ID NO: 32, and the HCDR3 comprises or consists of an amino acid sequence of SEQ ID NO: 33; the second light chain comprises LCDR1, LCDR2, and LCDR3, wherein the LCDR1 comprises or consists of an amino acid sequence of SEQ ID NO: 36, the LCDR2 comprises or consists of an amino acid sequence of SEQ ID NO: 37, and the LCDR3 comprises or consists of an amino acid sequence of SEQ ID NO: 38;
2) the first light chain comprises LCDR1, LCDR2, and LCDR3, wherein the LCDR1 comprises or consists of an amino acid sequence of SEQ ID NO: 6, the LCDR2 comprises or consists of an amino acid sequence of SEQ ID NO: 7, and the LCDR3 comprises or consists of an amino acid sequence of SEQ ID NO: 8; the first heavy chain comprises HCDR1, HCDR2, and HCDR3, wherein the HCDR1 comprises or consists of an amino acid sequence of SEQ ID NO: 1, the HCDR2 comprises or consists of an amino acid sequence of SEQ ID NO: 2, and the HCDR3 comprises or consists of an amino acid sequence of SEQ ID NO: 3; the second heavy chain comprises HCDR1, HCDR2, and HCDR3, wherein the HCDR1 comprises or consists of an amino acid sequence of SEQ ID NO: 41, the HCDR2 comprises or consists of an amino acid sequence of SEQ ID NO: 42, and the HCDR3 comprises or consists of an amino acid sequence of SEQ ID NO: 43; the second light chain comprises LCDR1, LCDR2, and LCDR3, wherein the LCDR1 comprises or consists of an amino acid sequence of SEQ ID NO: 46, the LCDR2 comprises or consists of an amino acid sequence of SEQ ID NO: 47, and the LCDR3 comprises or consists of an amino acid sequence of SEQ ID NO: 48;
3) the first light chain comprises LCDR1, LCDR2, and LCDR3, wherein the LCDR1 comprises or consists of an amino acid sequence of SEQ ID NO: 16, the LCDR2 comprises or consists of an amino acid sequence of SEQ ID NO: 17, and the LCDR3 comprises or consists of an amino acid sequence of SEQ ID NO: 18; the first heavy chain comprises HCDR1, HCDR2, and HCDR3, wherein the HCDR1 comprises or consists of an amino acid sequence of SEQ ID NO: 11, the HCDR2 comprises or consists of an amino acid sequence of SEQ ID NO: 12, and the HCDR3 comprises or consists of an amino acid sequence of SEQ ID NO: 13; the second heavy chain comprises HCDR1, HCDR2, and HCDR3, wherein the HCDR1 comprises or consists of an amino acid sequence of SEQ ID NO: 31, the HCDR2 comprises or consists of an amino acid sequence of SEQ ID NO: 32, and the HCDR3 comprises or consists of an amino acid sequence of SEQ ID NO: 33; the second light chain comprises LCDR1, LCDR2, and LCDR3, wherein the LCDR1 comprises or consists of an amino acid sequence of SEQ ID NO: 36, the LCDR2 comprises or consists of an amino acid sequence of SEQ ID NO: 37, and the LCDR3 comprises or consists of an amino acid sequence of SEQ ID NO: 38;
4) the first light chain comprises LCDR1, LCDR2, and LCDR3, wherein the LCDR1 comprises or consists of an amino acid sequence of SEQ ID NO: 16, the LCDR2 comprises or consists of an amino acid sequence of SEQ ID NO: 17, and the LCDR3 comprises or consists of an amino acid sequence of SEQ ID NO: 18; the first heavy chain comprises HCDR1, HCDR2, and HCDR3, wherein the HCDR1 comprises or consists of an amino acid sequence of SEQ ID NO: 11, the HCDR2 comprises or consists of an amino acid sequence of SEQ ID NO: 12, and the HCDR3 comprises or consists of an amino acid sequence of SEQ ID NO: 13; the second heavy chain comprises HCDR1, HCDR2, and HCDR3, wherein the HCDR1 comprises or consists of an amino acid sequence of SEQ ID NO: 41, the HCDR2 comprises or consists of an amino acid sequence of SEQ ID NO: 42, and the HCDR3 comprises or consists of an amino acid sequence of SEQ ID NO: 43; the second light chain comprises LCDR1, LCDR2, and LCDR3, wherein the LCDR1 comprises or consists of an amino acid sequence of SEQ ID NO: 46, the LCDR2 comprises or consists of an amino acid sequence of SEQ ID NO: 47, and the LCDR3 comprises or consists of an amino acid sequence of SEQ ID NO: 48;
5) the first light chain comprises LCDR1, LCDR2, and LCDR3, wherein the LCDR1 comprises or consists of an amino acid sequence of SEQ ID NO: 26, the LCDR2 comprises or consists of an amino acid sequence of SEQ ID NO: 27, and the LCDR3 comprises or consists of an amino acid sequence of SEQ ID NO: 28; the first heavy chain comprises HCDR1, HCDR2, and HCDR3, wherein the HCDR1 comprises or consists of an amino acid sequence of SEQ ID NO: 21, the HCDR2 comprises or consists of an amino acid sequence of SEQ ID NO: 22, and the HCDR3 comprises or consists of an amino acid sequence of SEQ ID NO: 23; the second heavy chain comprises HCDR1, HCDR2, and HCDR3, wherein the HCDR1 comprises or consists of an amino acid sequence of SEQ ID NO: 31, the HCDR2 comprises or consists of an amino acid sequence of SEQ ID NO: 32, and the HCDR3 comprises or consists of an amino acid sequence of SEQ ID NO: 33; the second light chain comprises LCDR1, LCDR2, and LCDR3, wherein the LCDR1 comprises or consists of an amino acid sequence of SEQ ID NO: 36, the LCDR2 comprises or consists of an amino acid sequence of SEQ ID NO: 37, and the LCDR3 comprises or consists of an amino acid sequence of SEQ ID NO: 38;
   or
6) the first light chain comprises LCDR1, LCDR2, and LCDR3, wherein the LCDR1 comprises or consists of an amino acid sequence of SEQ ID NO: 26, the LCDR2 comprises or consists of an amino acid sequence of SEQ ID NO: 27, and the LCDR3 comprises or consists of an amino acid sequence of SEQ ID NO: 28; the first heavy chain comprises HCDR1, HCDR2, and HCDR3, wherein the HCDR1 comprises or consists of an amino acid sequence of SEQ ID NO: 21, the HCDR2 comprises or consists of an amino acid sequence of SEQ ID NO: 22, and the HCDR3 comprises or consists of an amino acid sequence of SEQ ID NO: 23; the second heavy chain comprises HCDR1, HCDR2, and HCDR3, wherein the HCDR1 comprises or consists of an amino acid sequence of SEQ ID NO: 41, the HCDR2 comprises or consists of an amino acid sequence of SEQ ID NO: 42, and the HCDR3 comprises or consists of an amino acid sequence of SEQ ID NO: 43; the second light chain comprises LCDR1, LCDR2, and LCDR3, wherein the LCDR1 comprises or consists of an amino acid sequence of SEQ ID NO: 46, the LCDR2 comprises or consists of an amino acid sequence of SEQ ID NO: 47, and the LCDR3 comprises or consists of an amino acid sequence of SEQ ID NO: 48.

In another embodiment, the anti-CD79b×CD3 bispecific antibody provided herein comprises a first light chain, a first heavy chain, a second heavy chain, and a second light chain, wherein the first light chain comprises a VL set forth in SEQ ID NO: 9, 19, or 29, the first heavy chain comprises a VH set forth in SEQ ID NO: 4, 14, or 24, the second heavy chain comprises a VH set forth in SEQ ID NO: 34 or 44, and the second light chain comprises a VL set forth in SEQ ID NO: 39 or 49.

In a specific embodiment, the anti-CD79b×CD3 bispecific antibody provided herein comprises a first light chain, a first heavy chain, a second heavy chain, and a second light chain, wherein:
1) the first light chain comprises a VL set forth in SEQ ID NO: 9, the first heavy chain comprises a VH set forth in SEQ ID NO: 4, the second heavy chain comprises a VH set forth in SEQ ID NO: 34, and the second light chain comprises a VL set forth in SEQ ID NO: 39;
2) the first light chain comprises a VL set forth in SEQ ID NO: 9, the first heavy chain comprises a VH set forth in SEQ ID NO: 4, the second heavy chain comprises a VH set forth in SEQ ID NO: 44, and the second light chain comprises a VL set forth in SEQ ID NO: 49;
3) the first light chain comprises a VL set forth in SEQ ID NO: 19, the first heavy chain comprises a VH set forth in SEQ ID NO: 14, the second heavy chain comprises a VH set forth in SEQ ID NO: 34, and the second light chain comprises a VL set forth in SEQ ID NO: 39;
4) the first light chain comprises a VL set forth in SEQ ID NO: 19, the first heavy chain comprises a VH set forth in SEQ ID NO: 14, the second heavy chain comprises a VH set forth in SEQ ID NO: 44, and the second light chain comprises a VL set forth in SEQ ID NO: 49;
5) the first light chain comprises a VL set forth in SEQ ID NO: 29, the first heavy chain comprises a VH set forth in SEQ ID NO: 24, the second heavy chain comprises a VH set forth in SEQ ID NO: 34, and the second light chain comprises a VL set forth in SEQ ID NO: 39;
   or
6) the first light chain comprises a VL set forth in SEQ ID NO: 29, the first heavy chain comprises a VH set forth in SEQ ID NO: 24, the second heavy chain comprises a VH set forth in SEQ ID NO: 44, and the second light chain comprises a VL set forth in SEQ ID NO: 49.

In another embodiment, the anti-CD79b×CD3 bispecific antibody provided herein comprises a first light chain, a first heavy chain, a second heavy chain, and a second light chain, wherein the first heavy chain and the second heavy chain comprise the same or different Fc regions. In another preferred embodiment, the Fc regions contained in the first heavy chain and the second heavy chain have "knob" and "hole" structures, respectively, which interact to stabilize the spatial structure of the bispecific antibody.

In a specific embodiment, in the anti-CD79b×CD3 bispecific antibody described herein, the variable region contained in the first heavy chain and the second heavy chain is either homologous or heterologous to the Fc region. In another embodiment, the variable region contained in the first heavy chain and the second heavy chain is linked to the Fc region directly or via a linker. In a specific embodiment, the linker is a flexible linker commonly used in the art. In another embodiment, the linker is (G4S)n, wherein n = 1-6, and preferably n =1, 2, 3, or 4.

In another aspect, the present invention provides an anti-CD79b×CD3 bispecific antibody, which comprises a first light chain, a first heavy chain, a second heavy chain, and a second light chain, wherein:
1) the first light chain comprises a sequence having at least 90%, 91%, 92%, 93%, 94%, 95%, 96%, 97%, 98%, 99%, or higher sequence identity to SEQ ID NO: 10 or consists of SEQ ID NO: 10; the first heavy chain comprises a sequence having at least 90%, 91%, 92%, 93%, 94%, 95%, 96%, 97%, 98%, 99%, or higher sequence identity to SEQ ID NO: 5 or consists of SEQ ID NO: 5; the second heavy chain comprises a sequence having at least 90%, 91%, 92%, 93%, 94%, 95%, 96%, 97%, 98%, 99%, or higher sequence identity to SEQ ID NO: 35 or consists of SEQ ID NO: 35; the second light chain comprises a sequence having at least 90%, 91%, 92%, 93%, 94%, 95%, 96%, 97%, 98%, 99%, or higher sequence identity to SEQ ID NO: 40 or consists of SEQ ID NO: 40;
2) the first light chain comprises a sequence having at least 90%, 91%, 92%, 93%, 94%, 95%, 96%, 97%, 98%, 99%, or higher sequence identity to SEQ ID NO: 10 or consists of SEQ ID NO: 10; the first heavy chain comprises a sequence having at least 90%, 91%, 92%, 93%, 94%, 95%, 96%, 97%, 98%, 99%, or higher sequence identity to SEQ ID NO: 5 or consists of SEQ ID NO: 5; the second heavy chain comprises a sequence having at least 90%, 91%, 92%, 93%, 94%, 95%, 96%, 97%, 98%, 99%, or higher sequence identity to SEQ ID NO: 45 or consists of SEQ ID NO: 45; the second light chain comprises a sequence having at least 90%, 91%, 92%, 93%, 94%, 95%, 96%, 97%, 98%, 99%, or higher sequence identity to SEQ ID NO: 50 or consists of SEQ ID NO: 50;
3) the first light chain comprises a sequence having at least 90%, 91%, 92%, 93%, 94%, 95%, 96%, 97%, 98%, 99%, or higher sequence identity to SEQ ID NO: 20 or consists of SEQ ID NO: 20; the first heavy chain comprises a sequence having at least 90%, 91%, 92%, 93%, 94%, 95%, 96%, 97%, 98%, 99%, or higher sequence identity to SEQ ID NO: 15 or consists of SEQ ID NO: 15; the second heavy chain comprises a sequence having at least 90%, 91%, 92%, 93%, 94%, 95%, 96%, 97%, 98%, 99%, or higher sequence identity to SEQ ID NO: 35 or consists of SEQ ID NO: 35; the second light chain comprises a sequence having at least 90%, 91%, 92%, 93%, 94%, 95%, 96%, 97%, 98%, 99%, or higher sequence identity to SEQ ID NO: 40 or consists of SEQ ID NO: 40;
4) the first light chain comprises a sequence having at least 90%, 91%, 92%, 93%, 94%, 95%, 96%, 97%, 98%, 99%, or higher sequence identity to SEQ ID NO: 20 or consists of SEQ ID NO: 20; the first heavy chain comprises a sequence having at least 90%, 91%, 92%, 93%, 94%, 95%, 96%, 97%, 98%, 99%, or higher sequence identity to SEQ ID NO: 15 or consists of SEQ ID NO: 15; the second heavy chain comprises a sequence having at least 90%, 91%, 92%, 93%, 94%, 95%, 96%, 97%, 98%, 99%, or higher sequence identity to SEQ ID NO: 45 or consists of SEQ ID NO: 45; the second light chain comprises a sequence having at least 90%, 91%, 92%, 93%, 94%, 95%, 96%, 97%, 98%, 99%, or higher sequence identity to SEQ ID NO: 50 or consists of SEQ ID NO: 50;
5) the first light chain comprises a sequence having at least 90%, 91%, 92%, 93%, 94%, 95%, 96%, 97%, 98%, 99%, or higher sequence identity to SEQ ID NO: 30 or consists of SEQ ID NO: 30; the first heavy chain comprises a sequence having at least 90%, 91%, 92%, 93%, 94%, 95%, 96%, 97%, 98%, 99%, or higher sequence identity to SEQ ID NO: 25 or consists of SEQ ID NO: 25; the second heavy chain comprises a sequence having at least 90%, 91%, 92%, 93%, 94%, 95%, 96%, 97%, 98%, 99%, or higher sequence identity to SEQ ID NO: 35 or consists of SEQ ID NO: 35; the second light chain comprises a sequence having at least 90%, 91%, 92%, 93%, 94%, 95%, 96%, 97%, 98%, 99%, or higher sequence identity to SEQ ID NO: 40 or consists of SEQ ID NO: 40;
   or
6) the first light chain comprises a sequence having at least 90%, 91%, 92%, 93%, 94%, 95%, 96%, 97%, 98%, 99%, or higher sequence identity to SEQ ID NO: 30 or consists of SEQ ID NO: 30; the first heavy chain comprises a sequence having at least 90%, 91%, 92%, 93%, 94%, 95%, 96%, 97%, 98%, 99%, or higher sequence identity to SEQ ID NO: 25 or consists of SEQ ID NO: 25; the second heavy chain comprises a sequence having at least 90%, 91%, 92%, 93%, 94%, 95%, 96%, 97%, 98%, 99%, or higher sequence identity to SEQ ID NO: 45 or consists of SEQ ID NO: 45; the second light chain comprises a sequence having at least 90%, 91%, 92%, 93%, 94%, 95%, 96%, 97%, 98%, 99%, or higher sequence identity to SEQ ID NO: 50 or consists of SEQ ID NO: 50.

In one embodiment, the light chain variable domain of the first light chain and the heavy chain variable domain of the first heavy chain pair to form an antigen recognition site of CD79b, and the heavy chain variable domain of the second heavy chain and the light chain variable domain of the second light chain pair to form an antigen recognition site of CD3. The Fc domains of each of the first heavy chain and the second heavy chain interact to form an Fc region. In a specific embodiment, the Fc domains of each of the first heavy chain and the second heavy chain contain a mutation that stabilizes the interaction, e.g., contain a "knob-in-hole" mutation.

In one aspect, the present invention also provides a polynucleotides (nucleic acid) encoding the anti-CD79b×CD3 bispecific antibody of the present invention, and a vector comprising the polynucleotides, wherein the vector is preferably an expression vector.

In another aspect, the present invention provides a host cell comprising the polynucleotide or the vector of the present invention. The present invention also provides a method for producing the anti-CD79b×CD3 bispecific antibody of the present invention, which comprises the following steps: (i) culturing the host cell of the present invention under conditions suitable for expressing the anti-CD79b×CD3 bispecific antibody of the present invention, and (ii) recovering the anti-CD79b×CD3 bispecific antibody of the present invention.

In one aspect, the present invention provides a diagnostic kit and a pharmaceutical composition comprising the anti-CD79b×CD3 bispecific antibody of the present invention. Further, provided is use of the anti-CD79b×CD3 bispecific antibody, the diagnostic kits, or the pharmaceutical compositions of the present invention in the treatment, prevention, and/or diagnosis of a disease related to CD79b activity, in particular in the treatment, prevention, and/or diagnosis of non-Hodgkin's lymphoma.

In another aspect, the present invention provides a method for treating a disease related to CD79b activity, which comprises administering to a patient in need thereof a therapeutically effective amount of the anti-CD79b×CD3 bispecific antibody of the present invention or the pharmaceutical composition of the present invention. Preferably, the disease is cancer that overexpresses CD79b; more preferably, the disease is non-Hodgkin's lymphoma. Unless otherwise defined, all technical and scientific terms used herein have the same meaning as commonly understood by those of ordinary skill in the art to which the present invention belongs. All publications, patent applications, patents, and other references mentioned herein are incorporated by reference in their entireties. In addition, the materials, methods, and examples described herein are illustrative only and are not intended to be limiting. Other features, objectives, and advantages of the present invention will be apparent from the specification and drawings, and from the appended claims.

### BRIEF DESCRIPTION OF THE DRAWINGS

The preferred embodiments of the present invention described in detail below will be better understood when read in conjunction with the following drawings. For the purpose of illustrating the present invention, currently, preferred embodiments are shown in the drawings. However, it should be understood that the present invention is not limited to an accurate arrangement and means of the embodiments shown in the drawings.
FIG. 1 shows the binding ability of chimeric anti-CD79b antibodies for Ramos cells.
FIG. 2 shows the binding ability of chimeric anti-CD79b antibodies for BJAB cells.
FIG. 3 shows the binding ability of humanized anti-CD79b antibodies for Ramos cells.
FIG. 4 shows the binding ability of humanized anti-CD79b antibodies for BJAB cells.
FIG. 5 shows the detection of ADCC activity of humanized anti-CD79b antibodies.
FIG. 6 shows the binding ability of exemplary antibodies for BJAB cells.
FIG. 7 shows the binding ability of exemplary antibodies for WSU-DLCL2 cells.
FIG. 8 shows the activation of NFAT signals mediated by exemplary antibodies.
FIG. 9 shows the killing of tumor cells by human CD8+ T cells mediated by exemplary antibodies.
FIG. 10 shows the flow cytometry assay of the activation of human CD8+ T cells by exemplary antibodies.
FIG. 11 shows the flow cytometry assay of the amount of cytokine IFN-γ released during the killing of tumor cells by human CD8+ T cells induced by exemplary antibodies.
FIG. 12 shows the flow cytometry assay of the amount of cytokine TNFα released during the killing of tumor cells by human CD8+ T cells induced by exemplary antibodies.
FIG. 13 shows the flow cytometry assay of the activation of human CD4+ T cells by exemplary antibodies.
FIG. 14. shows the CD8+ T cell proliferation promoted by exemplary antibodies.
FIG. 15. shows the CD4+ T cell proliferation promoted by exemplary antibodies.
FIG. 16 shows the tumor inhibitory effect of exemplary antibodies in WSU-DLCL2 tumor-bearing humanized mouse models.
FIG. 17 shows the tumor inhibitory effect of exemplary antibodies in Ramos tumor-bearing humanized mouse models.
FIG. 18 shows the PK of bispecific antibodies in mice.

### DETAILED DESCRIPTION

### I. Definitions

Before the present invention is described in detail below, it should be understood that the present invention is not limited to the particular methodology, protocols, and reagents described herein, as these may vary. It should also be understood that the terminology used herein is only intended to describe specific embodiments rather than limit the scope of the present invention, which will be limited only by the appended claims. Unless otherwise defined, any technical and scientific term used herein has the same meaning as commonly understood by those of ordinary skill in the art to which the present invention belongs.

For the purpose of explaining this specification, the following definitions will be used, and wherever appropriate, terms used in the singular form may also include the plural form, and vice versa. It should be understood that the terms used herein are for the purpose of describing specific embodiments only, and are not intended to be limiting. The term "about" used in combination with a numerical value is intended to encompass the numerical values in a range from a lower limit less than the specified numerical value by 5% to an upper limit greater than the specified numerical value by 5%.

The term "and/or" means that when used to connect two or more options, it should be understood to refer to any one of the options or any two or more of the options.

The term "comprise" or "include" means that the described elements, integers or steps are included, but not to the exclusion of any other elements, integers or steps. The term "comprise" or "include" used herein, unless otherwise specified, also encompasses the situation where the entirety consists of the described elements, integers or steps. For example, when referring to an antibody variable region "comprising" a specific sequence, it is also intended to encompass an antibody variable region consisting of the specific sequence.

The term "antibody" is used herein in the broadest sense and encompasses a variety of antibody structures, including but not limited to, a monoclonal antibody, a polyclonal antibody, a recombinant antibody, a humanized antibody, a chimeric antibody, a multispecific antibody (e.g., a bispecific antibody), a single-chain antibody, an intact antibody, or an antibody fragment thereof that exhibits the desired antigen-binding activity. An intact antibody will generally comprise at least two full-length heavy chains and two full-length light chains, but may comprise fewer chains in some cases; for example, natural antibodies in a camel may only comprise heavy chains.

The term "antigen-binding fragment" (used interchangeably herein with "antibody fragment" and "antigen-binding portion") refers to a molecule different from an intact antibody, which comprises a portion of the intact antibody and binds to an antigen to which the intact antibody binds. Examples of antigen-binding fragments include, but are not limited to, an Fv, a Fab, a Fab', a Fab'-SH, a F(ab')₂; diabodies (dAbs); linear antibodies; single-chain antibodies (e.g., scFvs); single-domain antibodies; antigen-binding fragments of bivalent or bispecific antibodies; camelid antibodies; and other fragments that exhibit the desired ability to bind to an antigen (e.g., CD79b and/or CD3).

As used herein, the term "bind" and "specific bind" mean that the binding effect of an antibody is selective for antigens and can be distinguished from unwanted or non-specific interactions. The ability of an antibody to bind to a particular antigen can be determined by an enzyme-linked immunosorbent assay (ELISA), surface plasmon resonance (SPR), or bio-layer interferometry (ForteBio), or a conventional binding assay known in the art. For example, if an antibody binds to CD79b and/or CD3 with a KD of about 1×10⁻⁷ or less, a KD of about 1×10⁻⁸ or less, a KD of about 1×10⁻⁹ or less, a KD of about 1×10⁻¹⁰ or less, or a KD of about 1×10⁻¹¹ or less in SPR, it is the antibody that "specifically binds to CD79b and/or CD3". However, an antibody that specifically binds to CD79b and/or CD3 may have cross-reactivity with a CD79b and/or CD3 protein from other species. For example, an antibody specific to human CD79b and/or CD3, in some embodiments, can cross-react with cynomolgus monkey CD79b and/or CD3. A method for determining cross-reactivity includes the method described in examples and standard assays known in the art, such as biological optical interferometry or flow cytometry.

The term "single-chain variable fragment" or "scFv" is a small-molecule genetically engineered antibody. It is a small-molecule recombinant antibody obtained by connecting (usually via a synthetic linker peptide (or a linker)) a heavy chain variable region (VH) with a light chain variable region (VL) of a natural antibody at the DNA level by genetic engineering. Compared with an intact antibody molecule, the single-chain scFv antibody has the following advantages: having the variable regions of an intact antibody, thereby retaining the antigen specificity and antigen-binding activity of the original antibody; having no Fc region, and thus having weak immunogenicity and barely producing immunoreaction when used in humans; easy-to-operate and suitable for use as a genetically engineered component in preparing other antigen-specific binding molecules with new properties, such as full-length antibodies, scFv-Fc antibodies, etc.

The term "Fc region" is used herein to define a C-terminus region of an immunoglobulin heavy chain, which comprises at least a portion of a constant region. The term includes Fc regions of native sequences and variant Fc regions. In certain embodiments, a human IgG heavy chain Fc region generally extends from Cys226 or Pro230 to the carbonyl terminus of a heavy chain. However, the C-terminal lysine (Lys447) of the Fc region may or may not be present. Unless otherwise stated, amino acid residues in the Fc region or constant region are numbered according to the EU numbering system, also called the EU index, as described in Kabat et al., Sequences of Proteins of Immunological Interest, 5th Ed. Public Health Service, National Institutes of Health, Bethesda, MD, 1991.

The term "knob-in-hole" means that a "knob" structure is created on one Fc chain of the bispecific antibody molecule described herein, and a "hole" structure is created on the other chain. As such, the hole and the knob are of the same or similar size and are suitably positioned such that upon interaction of two Fcs, the knob of one Fc can be positioned in the corresponding hole of the other Fc, thereby stabilizing the structure of the heteromultimer (see, e.g., U.S. Pat. No: 5,731,168).

In some embodiments, the knob can be constructed by substituting a small amino acid side chain with a larger side chain, according to the prior art in the field. In some embodiments, the hole can be constructed by substituting a large amino acid side chain with a smaller side chain.

The term "variable region" or "variable domain" refers to a domain of a heavy chain or light chain of an antibody involved in the binding of the antibody to an antigen. Variable domains of heavy and light chains of natural antibodies typically have similar structures, wherein each domain comprises four conserved framework regions (FRs) and three complementarity determining regions (see, e.g., Kindt et al., Kuby Immunology, 6th ed., W. H. Freeman and Co., page 91 (2007)). A single VH or VL domain may be sufficient to provide antigen-binding specificity.

"Complementarity-determining region" or "CDR region" or "CDR" or "highly variable region" is a region in an antibody variable domain that is highly variable in sequence, forms a structurally defined loop ("a hypervariable loop") and/or comprises antigen-contacting residues ("antigen-contacting sites"). CDRs are primarily responsible for binding to antigen epitopes. The CDRs of heavy and light chains are numbered sequentially from the N-terminus and are generally referred to as CDR1, CDR2, and CDR3. The CDRs located in a heavy chain variable domain of an antibody are also referred to as HCDR1, HCDR2, and HCDR3, whereas the CDRs located in a light chain variable domain of an antibody are referred to as LCDR1, LCDR2, and LCDR3. In a given amino acid sequence of a light chain variable region or a heavy chain variable region, the CDR sequence thereof may be determined using a variety of schemes well known in the art, for example, Chothia based on the three-dimensional structure of antibodies and the topology of the CDR loops (Chothia et al., (1989) Nature 342: 877-883; Al-Lazikani et al., "Standard conformations for the canonical structures of immunoglobulins", Journal of Molecular Biology, 273: 927-948 (1997)), Kabat based on antibody sequence variability (Kabat et al., Sequences of Proteins of Immunological Interest, 4th edition, U.S. Department of Health and Human Services, National Institutes of Health (1987)), AbM (University of Bath), Contact (University College London), International ImMunoGeneTics database (IMGT) (imgt.cines.fr/ on the World Wide Web), and North CDR definition based on the affinity propagation clustering using a large number of crystal structures (North et al., "A New Clustering of Antibody CDR Loop Conformations", Journal of Molecular Biology, 406, 228-256, (2011)).

For example, Kabat- and Chothia-numbered CDR regions have different definition ranges.

| CDR | Kabat scheme | AbM scheme | Chothia scheme | Contact scheme | IMGT scheme |
|---|---|---|---|---|---|
| LCDR1 (Kabat and Chothia numbering systems) | L24-L34 | L24-L34 | L26-L32 | L30-L36 | L27-L32 |
| LCDR2 (Kabat and Chothia numbering systems) | L50-L56 | L50-L56 | L50-L52 | L46-L55 | L50-L52 |
| LCDR3 (Kabat and Chothia numbering systems) | L89-L97 | L89-L97 | L91-L96 | L89-L96 | L89-L96 |
| HCDR1 (Kabat numbering system) | H31-H35B | H26-H35B | H26-H32 | H30-H35B | H26-H35B |
| HCDR1 (Chothia numbering system) | H31-H35 | H26-H35 | H26-H32 | H30-H35 | H26-H35 |
| HCDR2 (Kabat and Chothia numbering systems) | H50-H65 | H50-H58 | H53-H55 | H47-H58 | H51-H57 |
| HCDR3 (Kabat and Chothia numbering systems) | H95-H102 | H95-H102 | H96-H101 | H93-H101 | H93-H102 |

Unless otherwise stated, the term "CDR" or "CDR sequence" used herein encompasses CDR sequences determined by any one of the schemes described above.

CDRs can also be determined based on having the same Kabat numbering positions as the reference CDR sequences. Unless otherwise stated, residue positions of an antibody variable region (including heavy chain variable region residues and light chain variable region residues) in the present invention are positions numbered according to the Kabat numbering system (Kabat et al., Sequences of Proteins of Immunological Interest, 5th Ed. Public Health Service, National Institutes of Health, Bethesda, Md. (1991)).

However, it should be noted that boundaries of CDRs of variable regions of an antibody based on different assignment systems may differ. That is, CDR sequences of variable regions of the same antibody defined by different assignment systems differ. Therefore, when it comes to defining an antibody with specific CDR sequences defined in the present invention, the scope of antibody also encompasses such antibodies whose variable region sequences comprise the specific CDR sequences, but having claimed CDR boundaries different from the specific CDR boundaries defined by the present invention due to a different protocol (e.g., different assignment system rules or their combinations) applied.

Antibodies with different specificities (i.e., different binding sites for different antigens) have different CDRs. However, although CDRs vary from antibody to antibody, only a limited number of amino acid positions within the CDRs are directly involved in antigen binding. The smallest overlapping region can be determined using at least two of the Kabat, Chothia, AbM, Contact, and North methods, thereby providing a "minimal binding unit" for antigen binding. The minimal binding unit may be a sub-portion of the CDR. As will be clear to those skilled in the art, residues in the remaining portions of the CDR sequences can be determined by the structure and protein folding of the antibody. Therefore, variants of any CDR presented herein are also considered. For example, in a variant of one CDR, the amino acid residue of the smallest binding unit may remain unchanged, while the remaining CDR residues defined according to Kabat or Chothia may be replaced by conservative amino acid residues.

The term "cytotoxic agent" used herein refers to a substance that inhibits or prevents cell functions and/or causes cell death or cell destruction.

The term "chemotherapeutic agent" includes chemical compounds useful in the treatment of cancer.

The term "small molecule drug" refers to a low molecular weight organic compound capable of regulating biological processes. "Small molecule" is defined as a molecule with a molecular weight of less than 10 kD, usually less than 2 kD, and preferably less than 1 kD. The small molecule includes but is not limited to inorganic molecules, organic molecules, organic molecules containing inorganic components, molecules containing radioactive atoms, synthetic molecules, peptide mimetics, and antibody mimetics. As therapeutic agents, small molecules penetrate cells better, are less susceptible to degradation and are less likely to induce an immune response compared with large molecules. The term "functional Fc region" refers to an Fc region that possesses the "effector functions" of Fc regions of native sequences. Exemplary "effector functions" include C1q binding, CDC, Fc receptor binding, ADCC, phagocytosis, cell surface receptor (e.g., B cell receptor, or BCR) down-regulation, and the like. Such effector functions generally require that the Fc region is associated with a binding domain (e.g., an antibody variable domain) and can be assessed using a variety of assays, such as those disclosed herein.

The term "therapeutic agent" described herein encompasses any substance effective in preventing or treating tumors (e.g., cancer), including chemotherapeutic agents, cytotoxic agents, vaccines, other antibodies, anti-infective active agents, small molecule drugs, or immunomodulatory agents.

The term "immunomodulatory agent" used herein refers to a natural or synthetic active agent or drug that suppresses or modulates an immune response. The immune response may be a humoral response or a cellular response.

The term "effective amount" refers to an amount or dosage of the antibody, fragment thereof, conjugate or composition of the present invention which generates expected effects in a patient in need of treatment or prevention after administration to the patient in a single or multiple doses. For therapeutic or prophylactic purposes, the "effective amount" can be divided into a "therapeutically effective amount" and a "prophylactically effective amount". The effective amount can be easily determined by an attending physician as a person skilled in the art by considering a variety of factors as follows: such as the species, size, age and general health of the mammal, the specific disease involved, the degree or severity of the disease, response in an individual patient, specific antibody administered, mode of administration, bioavailability profile of the administered formulation, selected administration regimen, and use of any concomitant therapy.

In one embodiment, an effective amount of the bispecific antibody of the present invention preferably inhibits a measurable parameter (e.g., tumor growth rate, tumor volume, etc.) by at least about 20%, more preferably at least about 40%, even more preferably at least about 50%, 60%, or 70%, and still more preferably at least about 80% or 90%, compared with a control.

The terms "host cell", "host cell line" and "host cell culture" are used interchangeably and refer to cells into which exogenous nucleic acids are introduced, including progenies of such cells. Host cells include "transformants" and "transformed cells", which include original primary transformed cells and progenies derived therefrom, regardless of the number of passages. Progeny may not be exactly the same as parent cells in terms of nucleic acid content, and may comprise mutations. Mutant progenies having the same function or biological activities that are screened or selected from the initially transformed cells are included herein.

As used herein, the term "multispecific" antibody refers to an antibody having at least two different antigen-binding sites, each of which binds to a different epitope of the same antigen or a different epitope of a different antigen. A multispecific antibody is an antibody having binding specificities for at least two different epitopes. In one embodiment, provided herein is a bispecific antibody having binding specificities for a first antigen target (CD79b) and a second antigen target (CD3). Given that the antibody construct according to the present invention is (at least) bispecific, it is not naturally occurring and it is clearly different from naturally occurring products. Thus, a "bispecific" antibody or immunoglobulin is an artificial hybrid antibody or immunoglobulin having at least two different binding sides with different specificities.

The term "target" means CD79b or CD3. The term "first target and second target" means that CD79b is the first target and CD3 is the second target or vice versa.

The term "cytokine" is a generic term for proteins that are released by a cell population and act as intercellular mediators on another cell. Examples of such cytokines are lymphokines; monokines; interleukins (IL), such as IL-1, IL-1α, IL-2, IL-3, IL-4, IL-5, IL-6, IL-7, IL-8, IL-9, IL-11, IL-12, and IL-15; tumor necrosis factors, such as TNF-α or TNF-β; and other polypeptide factors, including LIF and kit ligands (KL) and γ-interferons. As used herein, the term "cytokine" includes proteins from natural sources or from recombinant cell cultures and biologically active equivalents of native sequence cytokines, including small molecule entities produced by artificial synthesis, and pharmaceutically acceptable derivatives and salts thereof.

The term "immunoconjugate" is an antibody conjugated to one or more other substances, including but not limited to cytotoxic agents or labels.

The term "individual" or "subject" includes mammals. The mammals include, but are not limited to, domestic animals (e.g., cattle, goats, cats, dogs, and horses), primates (e.g., human and non-human primates such as monkeys), rabbits, and rodents (e.g., mice and rats). In some embodiments, the individual or subject is a human. The term "isolated" antibody is an antibody that has been separated from components of its natural environment. In some embodiments, the antibody is purified to a purity greater than 95% or 99% as determined by, e.g., electrophoresis (e.g., SDS-PAGE, isoelectric focusing (IEF) and capillary electrophoresis) or chromatography (e.g., ion exchange or reverse-phase HPLC). For a review of methods for assessing antibody purity, see, e.g., Flatman et al., J. Chromatogr., B848: 79-87 (2007).

The calculation of sequence identity between sequences is performed as follows.

To determine the percent identity of two amino acid sequences or two nucleic acid sequences, the sequences are aligned for optimal comparison purposes (e.g., for optimal alignment, gaps can be introduced in one or both of the first and second amino acid sequences or nucleic acid sequences, or non-homologous sequences can be discarded for comparison). In one preferred embodiment, the length of a reference sequence aligned for comparison purposes is at least 30%, at least 40%, at least 50% or 60%, and even more preferably at least 70%, 80%, 90%, or 100% of the length of the reference sequence. Amino acid residues or nucleotides at corresponding amino acid positions or nucleotide positions are then compared. When a position in the first sequence is occupied by the same amino acid residue or nucleotide at the corresponding position in the second sequence, the molecules are identical at this position.

A mathematical algorithm can be used to compare two sequences and calculate percent identity between the sequences. In one preferred embodiment, the percent identity between two amino acid sequences is determined with the Needlema and Wunsch algorithm ((1970) J. Mol. Biol., 48: 444-453; available at http://www.gcg.com) that has been integrated into the GAP program of the GCG software package, using the Blossum 62 matrix or PAM250 matrix and a gap weight of 16, 14, 12, 10, 8, 6, or 4 and a length weight of 1, 2, 3, 4, 5, or 6. In another preferred embodiment, the percent identity between two nucleotide acid sequences is determined with the GAP program of the GCG software package (available at http://www.gcg.com), using the NWSgapdna.CMP matrix and a gap weight of 40, 50, 60, 70, or 80 and a length weight of 1, 2, 3, 4, 5, or 6. A particularly preferred parameter set (and one that should be used unless otherwise stated) is a Blossum 62 scoring matrix with a gap penalty of 12, a gap extension penalty of 4, and a frameshift gap penalty of 5.

The percent identity between two amino acid sequences or nucleotide sequences can also be determined with a PAM120 weighted remainder table, a gap length penalty of 12, and a gap penalty of 4, using the E. Meyers and W Miller algorithm ((1989) CABIOS, 4:11-17) that has been incorporated into the ALIGN program (version 2.0). Additionally or alternatively, the nucleic acid sequences and protein sequences described herein can be further used as "query sequences" to perform searches against public databases to, e.g., identify other family member sequences or related sequences.

The term "pharmaceutical supplementary material" refers to diluents, adjuvants (e.g., Freund's adjuvants (complete and incomplete)), excipients, carriers, stabilizers, or the like, that are administered with the active substance.

The term "pharmaceutical composition" refers to a composition that exists in a form allowing effective biological activity of the active ingredient contained therein and does not contain additional ingredients having unacceptable toxicity to a subject to which the composition is administered.

The term "combination therapy" or "combined therapy" means that two or more therapeutic agents are administered to treat a cancer or an infection as described herein. Such administration includes co-administration of these therapeutic agents in a substantially simultaneous manner, for example, in a single capsule with a fixed proportion of active ingredients. Alternatively, such administration includes co-administration of the active ingredients in a variety of or separate containers (such as tablets, capsules, powder and liquid). The powder and/or liquid can be reconstituted or diluted to a desired dose before administration. In addition, such administration also includes using each type of the therapeutic agents at approximately the same time or in a sequential manner at different times. In any case, the therapeutic regimen will provide the beneficial effect of the pharmaceutical combination in the treatment of disorders or symptoms described herein.

As used herein, "treatment" (or "treat" or "treating") refers to slowing, interrupting, arresting, alleviating, stopping, lowering, or reversing the progression or severity of an existing symptom, disorder, condition, or disease.

As used herein, "prevention" (or "prevent" or "preventing") includes the inhibition of the development or progression of symptoms of a disease or disorder, or a specific disease or disorder. In some embodiments, subjects with family history of cancer are candidates for preventive regimens. Generally, in the context of cancer, the term "prevention" (or "prevent" or "preventing") refers to the administration of a drug prior to the onset of signs or symptoms of cancer, particularly in subjects at risk of cancer.

The term "vector" used herein refers to a nucleic acid molecule capable of proliferating another nucleic acid to which it is linked. The term includes vectors that serve as self-replicating nucleic acid structures as well as vectors binding to the genome of a host cell into which they have been introduced. Some vectors are capable of directing the expression of a nucleic acid to which they are operably linked. Such vectors are referred to as "expression vectors" herein.

### II. Antibody

The terms "CD79b", "target CD79b" and "human CD79b" as used herein refer to a CD79b recombinant protein.

The extracellular domain of CD79b consists of amino acids 29-159 according to UniProt. The "antibody against CD79b" and "anti-CD79b antibody" described herein refer to an antibody that specifically binds to CD79b. In one embodiment, an antibody that binds to CD79b has a dissociation constant (Kd) of 10⁻⁹ M or less, preferably 10⁻⁹ M to 10⁻¹³ M. In one embodiment, the anti-CD79b antibody binds to an epitope of CD79b that is conserved in CD79b from different species, preferably humans.

In one embodiment, the "anti-CD79b antibody" described herein comprises a heavy chain variable region comprising CDRs from SEQ ID NO: 4, 14, or 24 and a light chain variable region comprising CDRs from SEQ ID NO: 9, 19, or 29.

T cells or T lymphocytes are a class of lymphocytes that play a central role in cell-mediated immunity. The specificity of the T cell response is mediated by recognition of the antigen (displayed in the context of the major histocompatibility complex (MHC)) by the TCR. As part of the TCR, the CD3 receptor complex is a protein complex comprising a CD3γ (gamma) chain, a CD3δ (delta) chain, and two CD3ε (epsilon) chains present on the cell surface, and it is involved in the activation of cytotoxic T cells (CD8+ naive T cells) and T helper cells (CD4 + naive T cells). Clustering of CD3 on T cells, such as by immobilized anti-CD3 antibodies, results in T cell activation, which is similar to the engagement of the T cell receptor but independent of its clone-typical specificity.

It is found that CD3 binds to the membrane of all mature T cells. This high specificity and the presence of CD3 at various stages of T cell development make it a useful immunohistochemical marker for T cells in tissue sections. It is contemplated that the antibody construct according to the present invention generally and advantageously shows less non-specific T cell activation, which is not required in specific immunotherapy. This means that the risk of side effects is reduced.

The "anti-CD3 antibody" described herein refers to an antibody that binds to CD3. In one embodiment, the "anti-CD3 antibody" described herein comprises a heavy chain variable region comprising CDRs from SEQ ID NO: 34 or 44 and a light chain variable region comprising CDRs from SEQ ID NO: 39 or 49.

The terms "bispecific antibody against CD79b and CD3", "bispecific antibody specifically binding to CD79b and CD3", "anti-CD79b×CD3 bispecific antibody", "CD79b/CD3 bispecific antibody", and the like described herein refer to a bispecific antibody capable of binding to the targets CD79b and CD3 with sufficient affinity, and the bispecific antibody is capable of recruiting T cells and allowing redirected lysis of the target cells. The joined T cells are capable of continuous target cell lysis and are not affected by the immune escape mechanism interfering with peptide antigen processing and presentation or clonal T cell differentiation. In one embodiment, the "bispecific antibody against CD79b and CD3" described herein comprises a heavy chain variable region comprising CDRs from SEQ ID NO: 4, 14, or 24 and a light chain variable region comprising CDRs from SEQ ID NO: 9, 19, or 29 targeting CD79b and a heavy chain variable region comprising CDRs from SEQ ID NO: 34 or 44 and a light chain variable region comprising CDRs from SEQ ID NO: 39 or 49 targeting CD3. The antibody may be used as a diagnostic and/or therapeutic agent targeting cancer that expresses CD79b.

The anti-CD79b×CD3 bispecific antibody provided herein has the following advantages:
In some embodiments, the anti-CD79b×CD3 bispecific antibodies of the present invention, formed by assembling different anti-CD79b antibodies with anti-CD3 antibodies with different affinities, can facilitate the comprehensive assessment of the efficacy and safety of CD79b/CD3 bispecific antibodies at an early stage (*in vitro* screening phase) by introducing detection of the degree of T cell activation and the levels of the release of several cytokines in experiments of killing of tumor cells by CD8+T induced by the anti-CD79b×CD3 bispecific antibodies. Differential molecules with similar maximal killing and low cytokine release levels in the *in vitro* screening stage are selected for *in vivo* experiments and toxicity experiments, so that the risk of the cytokine storm can be reduced when such bispecific antibodies are used in clinical practice.

In some embodiments, the CD79b/CD3 bispecific antibody of the present invention simultaneously binds to CD79b on the surface of B-cell non-Hodgkin's lymphoma cells and CD3 on the surface of primary T cells and mediates the killing of CD79b-positive tumor cells by T cells. In some embodiments, the bispecific antibody of the present invention is capable of dose-dependently inducing killing by CD8+ T of B-cell non-Hodgkin's lymphoma cells with different levels of CD79b expression.

In some embodiments, the anti-CD79b×CD3 antibody of the present invention mediates the killing of human B-cell non-Hodgkin's lymphoma cells and dose-dependently activates CD8+ T cells and CD4+ T cells isolated from PBMCs. In some embodiments, the antibody of the present invention enhances the proliferation capacity of human CD8+ T and CD4+ T cells.

In some embodiments, the antibody of the present invention is effective in inhibiting tumor growth, and the tumor growth inhibition can be 67% or even 100% compared to controls.

In one embodiment of the present invention, anti-CD79b×CD3 bispecific antibodies having amino acid changes are encompassed herein, wherein the amino acid changes comprise replacements, insertions, or deletions of amino acids. Preferably, the amino acid change described herein is an amino acid replacement, preferably a conservative replacement.

In a preferred embodiment, the amino acid change described herein occurs in a region outside the CDR (e.g., in FR). More preferably, the amino acid change described herein occurs in a region outside the heavy chain variable region and/or outside the light chain variable region.

In some embodiments, the replacement is a conservative replacement. A conservative replacement refers to the replacement of an amino acid by another amino acid of the same class, e.g., the replacement of an acidic amino acid by another acidic amino acid, the replacement of a basic amino acid by another basic amino acid, or the replacement of a neutral amino acid by another neutral amino acid. Exemplary replacements are shown in table below:

| Original residue | Exemplary replacement | Preferred conservative amino acid replacement |
|---|---|---|
| Ala (A) | Val, Leu, Ile | Val |
| Arg (R) | Lys, Gln, Asn | Lys |
| Asn (N) | Gln, His, Asp, Lys, Arg | Gln |
| Asp (D) | Glu, Asn | Glu |
| Cys (C) | Ser, Ala | Ser |
| Gln (Q) | Asn, Glu | Asn |
| Glu (E) | Asp, Gln | Asp |
| Gly (G) | Ala | Ala |
| His (H) | Asn, Gln, Lys, Arg | Arg |
| Ile (I) | Leu, Val, Met, Ala, Phe, Nle | Leu |
| Leu (L) | Nle, Ile, Val, Met, Ala, Phe | Ile |
| Lys (K) | Arg, Gln, Asn | Arg |
| Met (M) | Leu, Phe, Ile | Leu |
| Phe (F) | Trp, Leu, Val, Ile, Ala, Tyr | Tyr |
| Pro (P) | Ala | Ala |
| Ser (S) | Thr | Thr |
| Thr (T) | Val, Ser | Ser |
| Trp (W) | Tyr, Phe | Tyr |
| Tyr (Y) | Trp, Phe, Thr, Ser | Phe |
| Val (V) | Ile, Leu, Met, Phe, Ala, Nle | Leu |

In certain embodiments, the replacement occurs in the CDRs of the antibody. Generally, the obtained variant has modifications (e.g., improvements) in certain biological properties (e.g., increased affinity) relative to the parent antibody and/or will substantially retain certain biological properties of the parent antibody. Exemplary replacement variants are affinity-matured antibodies.

In certain embodiments, the antibody provided herein is altered to increase or decrease the extent to which the antibody is glycosylated. Addition or deletion of glycosylation sites of an antibody can be conveniently achieved by altering the amino acid sequence to create or remove one or more glycosylation sites. When the antibody comprises an Fc region, carbohydrate attached thereto can be altered. In some applications, removing undesired modifications to glycosylation sites can be useful, for example, removing fucose modules to enhance antibody-dependent cell-mediated cytotoxicity (ADCC) (see Shield et al., (2002) JBC, 277:26733). In other applications, galactosidylation modification can be carried out to modify complement-dependent cytotoxicity (CDC).

In certain embodiments, one or more amino acid modifications may be introduced into an Fc region of an antibody provided herein, thus producing an Fc region variant. The Fc region variant may comprise a human Fc region sequence (such as human IgG1, IgG2, IgG3, or IgG4 Fc region) comprising an amino acid modification (such as replacement) at one or more amino acid positions. For examples of the Fc variant, see U.S. Patent No. 7,332,581, U.S. Patent No. 6,737,056, U.S. Patent No. 6,737,056; WO 2004/056312 and Shields et al., J. Biol. Chem., 9(2):6591-6604 (2001), U.S. Patent No. 6,194,551, WO 99/51642 and Idusogie et al., J. Immunol., 164:4178-4184 (2000), U.S. Patent No. 7,371,826, Duncan & Winter, Nature 322:738-40 (1988); U.S. Patent No. 5,648,260; U.S. Patent No. 5,624,821; and WO 94/29351.

In certain embodiments, antibodies modified by cysteine engineering may need to be produced, such as "sulfo-MAb", wherein one or more residues of the antibodies are replaced by cysteine residues. A cysteine-modified antibody can be produced as described, for example, in U.S. Patent No. 7,521,541.

In certain embodiments, the antibody provided herein can be further modified to comprise other non-protein portions known in the art and readily available. Suitable portions for antibody derivatization include, but are not limited to, water-soluble polymers. Non-limiting examples of water-soluble polymers include, but are not limited to, polyethylene glycol (PEG), ethylene glycol/propylene glycol copolymer, carboxymethyl cellulose, glucan, polyvinyl alcohol, polyvinylpyrrolidone, poly-1,3-dioxane, poly-1,3,6-trioxane, ethylene/maleic anhydride copolymer, polyamino acid (homopolymer or random copolymer), and glucan or poly(n-vinylpyrrolidone)polyethylene glycol, propylene glycol homopolymer, polypropylene oxide/ethylene oxide copolymer, polyoxyethylated polyol (such as glycerol), polyvinyl alcohol, and mixtures thereof.

### III. Nucleic acid of the present invention, and vector and host cell comprising same

In one aspect, the present invention provides a nucleic acid encoding any of the above anti-CD79b antibodies, anti-CD3 antibodies, and anti-CD79b×CD3 antibodies, or antigen-binding fragments thereof. The present invention also encompasses nucleic acids that hybridize to the nucleic acid described above under stringent conditions, nucleic acids that have one or more replacements (e.g., conservative replacements), deletions, or insertions compared with the nucleic acid described above, or nucleic acid sequences having at least 80%, at least 85%, at least 90%, 91%, 92%, 93%, 94%, 95%, 96%, 97%, 98%, 99%, or higher identity to the nucleic acid described above.

In another aspect, the present invention provides a vector comprising the nucleic acid described above. In a preferred embodiment, the vector is an expression vector. It is well understood by those skilled in the art that vectors commonly used in the art to which the present invention pertains may be applied to the present invention.

In one embodiment, the present invention provides a host cell comprising the nucleic acid or the vector. The suitable host cell for cloning or expressing the vector encoding the antibody includes prokaryotic cells or eukaryotic cells described herein. In one embodiment, the host cell is eukaryotic. In another embodiment, the host cell is selected from a yeast cell, a mammal cell (e.g., CHO cell or 293 cell), and other cells suitable for preparing an antibody or an antigen-binding fragment thereof.

### IV. Pharmaceutical composition

In some embodiments, the present invention provides a composition comprising any of the anti-CD79b×CD3 antibodies or the antigen-binding fragments thereof described herein, wherein preferably, the composition is a pharmaceutical composition. In one embodiment, the composition further comprises a pharmaceutical supplementary material. In one embodiment, the composition (e.g., the pharmaceutical composition) comprises the anti-CD79b×CD3 antibody or the antigen-binding fragment thereof of the present invention and a combination of one or more other therapeutic agents (e.g., chemotherapeutic agents, cytotoxic agents, vaccines, other antibodies, anti-infective active agents, small molecule drugs, or immunomodulatory agents).

The pharmaceutical composition of the present invention can further comprise one or more other active ingredients that are required for a specific indication being treated, preferably active ingredients that do not adversely affect activities of one another. For example, it may be also desirable to provide other anti-cancer active ingredients, such as chemotherapeutic agents, cytotoxic agents, vaccines, other antibodies, anti-infective active agents, small molecule drugs, or immunomodulatory agents. The active ingredients are suitably combined in an amount effective for an intended purpose.

### EXAMPLES

The following examples are described to assist in understanding the present invention. The examples are not intended to, and should not be construed as, limiting the protection scope of the present invention in any way, and various modifications may be made by those skilled in the art according to the description of the specification of the present application.

Unless otherwise indicated, conventional methods of chemistry, biochemistry, organic chemistry, molecular biology, microbiology, recombinant DNA techniques, genetics, immunology and cell biology that are known in the art will be employed for the implementation of the present invention. Descriptions of such methods can be found, for example, in Sambrook et al., Molecular Cloning: A Laboratory Manual (3rd Ed., 2001); Sambrook et al., Molecular Cloning: A Laboratory Manual (2nd Ed., 1989); Maniatis et al., Molecular Cloning: A Laboratory Manual (1982); Ausubel et al., Current Protocols in Molecular Biology (John Wiley and Sons, updated in July 2008); Short Protocols in Molecular Biology: A Compendium of Methods from Current Protocols in Molecular Biology, Greene Pub. Associates and Wiley-Interscience; Glover, DNA Cloning: A Practical Approach, vol. I&II (IRL Press, Oxford, 1985); Anand, Techniques for the Analysis of Complex Genomes, (Academic Press, New York, 1992); Transcription and Translation (B. Hames&S. Higgins, Eds., 1984); Perbal, A Practical Guide to Molecular Cloning (1984); Harlow and Lane, Antibodies (Cold Spring Harbor Laboratory Press, Cold Spring Harbor, N.Y., 1998); Current Protocols in Immunology (Q. E. Coligan, A. M. Kruisbeek, D. H. Margufies, E. M. Shevach and W. Strober, eds., 1991*); Annual Review of Immunology,* and journals and monographs such as *Advances in Immunology.*

### Molecular structure design of antibodies of the present invention

In one aspect, the present invention designs monospecific antibodies targeting CD79b, which can bind to CD79b on the surface of B-cell non-Hodgkin's lymphoma cells. Based on 5 anti-CD79b antibodies (34F1, 11G10, 38D9, 54H6, and 60D9), humanization is carried out to obtain 5 humanized anti-CD79b antibodies (hz34F1.14, hz11G10.9.p1, hz38D9B3.11, hz54H6A3.13, and hz60D9H6.2).

In another aspect, the present invention designs T-cell engager bispecific antibodies targeting CD79b and CD3 simultaneously, which can simultaneously bind to CD79b on the surface of B-cell non-Hodgkin's lymphoma cells and CD3 on the surface of T cells. Bispecific antibodies targeting both CD79b and CD3 were designed based on three anti-CD79b antibodies (38D9B3.11, 11G10.9.p1, and 34F1.14) and two anti-CD3 antibodies with different affinities (sp34.24 and sp34.87).

6 bispecific antibody molecules with a 1+1 format (38D9B3.11/sp34.87, 38D9B3.11/sp34.24, 11G10.9.p1/sp34.87, 11G10.9.p1/sp34.24, 34F1.14/sp34.87, and 34F1.14/sp34.24) are designed. The heavy chain mispairing of bispecific antibodies is solved using the Fc "knob-in-hole" technology, wherein Fc is the heavy chain constant region of IgG1; and moreover, amino acid mutations L234A and L235A (according to "EU" numbering of Kabat) that weaken the effector function are introduced.

### Example 1. Preparation of Hybridoma Cells

### Immunization

The CD79b protein (sino, Cat# 29750-H08H) was emulsified with Freund's complete adjuvant (Sigma, Cat# F5881) and then used to immunize Balb/c mice (purchased from Beijing Vital River Laboratory Animal Technology Co., Ltd.), and the CD79b protein was emulsified with Freund's incomplete adjuvant (Sigma, Cat# F5506) two weeks later and used to immunize the mice three times by intramuscular injection once every two weeks (50 µg of protein per mouse).

### Cell fusion

When the serum titer met the requirement, the spleen of a mouse was taken to prepare a B lymphocyte suspension. The B lymphocyte suspension was mixed with SP2/0 myeloma cells (ATCC) in a ratio of 1:2 to 1:1, and then electrofusion was performed. The fused cells were transferred from an electrode dish into a 50-mL centrifuge tube and diluted with an HAT-containing medium to 1-2 × 10⁴ cells/mL, and the cell suspension was added to a 96-well plate at 100 µL/well. The screening medium was replaced on day 7 after fusion, the resulting product was detected by flow cytometry (FACS) after 10 days (or longer, depending on the cell growth state) of culture, and positive clones were selected.

### Screening of positive clones of hybridoma cells (FACS)

Hybridoma cells specifically expressing an anti-CD79b antibody were selected by a flow cytometer (FACS). The cells to be detected (Ramos) were counted, diluted to 1 × 10⁶ cells/mL, and added to a U-shaped-bottom 96-well plate at 100 µL/well. The cells were centrifuged at 500 g for 5 min to remove the cell culture medium. The hybridoma culture supernatant in the 96-well plate was added to the U-shaped plate at 100 µL/well, the cells were resuspended, and the suspension was left to stand on ice for 30 min. The suspension was centrifuged at 500 g for 5 min to remove the supernatant, and the cells were washed once with PBS. PBS was removed by centrifugation at 500 g for 5 min, an FITC-labeled anti-mouse Fab secondary antibody (Jackson Immunoresearch, Cat# 115-545-006, 1:500 dilution in PBS) was added at 100 µL/well, and 100 µL of PE-labeled anti-human Fc secondary antibody (Biolegend, Cat# 409304) was added for the positive control antibody. The resulting mixture was incubated on ice in the dark for 30 min. The mixture was centrifuged at 500 g for 5 min to remove the supernatant, and the cells were washed once with PBS. The cells were resuspended with 50 µL of 1× PBS and the suspension was loaded for FACS assay. The positive clones were subcloned and monoclonal cells were picked out.

### Subcloning of positive hybridoma cells

Procedures of subcloning: a medium was added to a 96-well plate at 200 µL/well; compared with the screening medium, this medium had the same ingredients except that HAT was replaced with HT (Gibco, Cat# 11067-030). Cells in the positive wells selected by fusing were prepared into a cell suspension, the cell suspension was then added to the first row at 100 µL/well, then the cell suspension of the first row was added to the second row at 100 µL/well after being well mixed, and then the cell suspension of the second row was added to the next row at 100 µL/well after being well mixed; the above steps were repeated; the 96-well plate was left to stand for 30 min, and the cells were observed and counted under a microscope. A corresponding volume containing about 100 cells was added to 20 mL of medium for well mixing and plating at 200 µL/well. One week later, the cells were observed under a microscope, and monoclonal wells were identified and marked. When the cell confluence in each well reached not less than 50%, the cells were detected by a method the same as the FACS screening method described above to select target positive wells, and the affinity of the obtained hybridoma cell supernatant for the antigen was detected by using the bio-layer interferometry (ForteBio). All the detection results are shown in Table 1:

**Table 1. Detection results for hybridoma subclone supernatant**

| sample ID | Ramos(ratio) | KD(M) | | |
|---|---|---|---|---|
| | | huCD79b | cynoCD79b | huCD79b-N |
| 34F1 | 33.46 | 1.85E-09 | NB | NB |
| 11G10 | 54.49 | 1.92E-09 | NB | 3.18E-09 |
| 38D9 | 167.21 | 6.89E-10 | NB | 2.02E-09 |
| 54H6 | 88.49 | 2.06E-09 | NB | 6.55E-09 |
| 60D9 | 37.76 | 4.54E-09 | NB | NB |
| polmab | NA | 1.37E-08 | NB | 3.10E-09 |

### Example 2. Preparation of Recombinant Murine Antibodies

Antibody light and heavy chain gene sequences were extracted from the hybridoma candidate clones obtained in Example 1. About 5 × 10⁶ freshly-cultured cells of each strain were taken for RNA extraction (Macherey-Nagel, Cat# 740984.250). Reverse transcription was performed by using PrimeScript II 1st Strand cDNA Synthesis Kit (Takara) to obtain cDNA. An upstream primer was designed using a base sequence located in FR1 region at the 5' end and a downstream primer was designed using a base located in the antibody constant region or FR4 region, and then amplification was performed to obtain gene fragments of antibody light and heavy chain variable regions. The gene fragments were ligated into a T vector (Mighty TA-cloning Kit, Takara), monoclones were picked out for sequencing, and the sequencing results were analyzed and compared using MEGA7 software. Finally, the recombinant murine antibodies were constructed according to the specificity of the light and heavy chain variable region sequences of the antibodies and the affinity of the antibodies.

The gene fragments of the light and heavy chain variable regions of the antibodies were each ligated to a pcDNA3.1 vector by homologous recombinases from Nanjing Vazyme Biotech Co., Ltd. (Exnase^{®}II, Cat# C112-0 1), wherein an IgG1 subtype was selected as the constant region, and expression plasmids of antibody light and heavy chains were obtained.

The light chain plasmid and heavy chain plasmid of the same antibody were then mixed in a molar ratio of 1:1 and transfected into 293F cells by polyethyleneimine (PEI) (Polysciences, Cat# 23966). After 5-7 days of culture, the cell culture supernatant was collected and purified by a Protein A affinity column when the cell viability was below 60% to obtain a monoclonal antibody.

### Example 3. Binding Kinetics of Chimeric Antibodies of the Present Invention for Antigens as Determined by Bio-Layer Interferometry

The equilibrium dissociation constants (KD) for binding of the antibodies of the present invention to CD79b were determined by bio-layer interferometry (ForteBio). A ForteBio affinity assay of prior art was performed (Estep, P., et al., "High throughput solution Based measurement of antibody-antigen affinity and epitope binning", MAbs, 2013.5(2): 270-8).

Briefly, an AMQ (Pall, 1506091) sensor was equilibrated offline in an assay buffer for 30 min and then equilibrated online for 60 s to establish a baseline. The purified antibodies obtained as described above were each loaded online onto an AHQ sensor (ForteBio) for the ForteBio affinity assay. The sensor with the loaded antibody was then exposed to antigen CD79b before transferring the sensor to the assay buffer to measure the dissociation rate. The KD values were analyzed using ForteBio analysis software. The results of the antibody affinity assays are shown in Table 2:

**Table 2. Affinity (equilibrium dissociation constant KD) for binding of antigens and antibodies by ForteBio assay**

| **Sample ID** | KD (M) | | |
|---|---|---|---|
| | **huCD79b** | **huCD79b-N** | **huCD79b-C** |
| 34F1 | 4.68E-10 | 1.65E-09 | NB |
| 11G10 | 3.29E-10 | 9.92E-10 | NB |
| 38D9 | 4.65E-10 | 7.76E-10 | NB |
| 54H6 | 8.11E-10 | 1.13E-09 | NB |
| 60D9 | 4.31E-10 | 1.96E-09 | NB |
| A7v14b | 1.84E-09 | 8.56E-09 | NB |
| polmab | 1.27E-08 | 3.22E-09 | NB |

### Example 4. Binding Assays for Chimeric Antibodies and B-Cell Non-Hodgkin's Lymphoma Cell Lines

To verify whether the antibodies of the present invention can bind to an antigen expressed on the cell surface, this study detected the binding of the antibodies to Ramos cells and BJAB cells using flow cytometry, and the experimental process is as follows:
Ramos (ATCC, Cat# CRL-1596) and BJAB (AddexBio, USA, Cat# C0003016) were passaged according to the conventional procedures. Cells were counted after centrifugation and resuspension, adjusted to a density of 4 × 10⁶ cells/mL, poured into a loading tank, and seeded to a 96-well plate at 50 µL/well using a multichannel pipettor. 50 µL of the antibody sample diluted in gradient was added to the 50 µL of cells in each well, and the mixture was then incubated in an incubator at 5% CO₂ and 37 °C for 30 min. The mixture was centrifuged at 400 g for 5 min, then 200 µL of PBS was added, and the resulting mixture was centrifuged at 400 g for 5 min. This procedure was repeated 3 times. Goat anti-human IgG PE (1:200) was added, and the mixture was incubated for 30 min in the dark at room temperature. The mixture was washed with 200 µL of PBS twice, centrifuged, and then resuspended in 100 µL of PBS, and the values were read using a flow cytometer. The values were fitted to obtain a curve and EC₅₀ values were calculated.

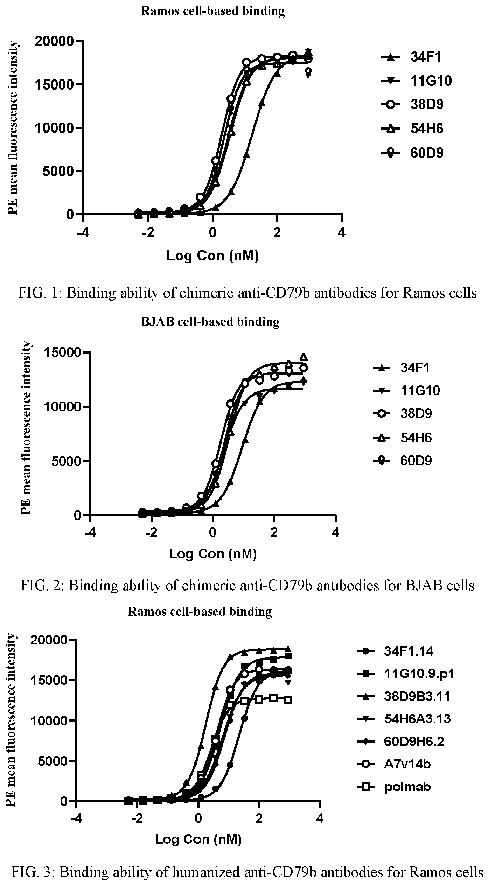

In experiments performed using the assay described above, the binding of the chimeric antibodies to CD79b on the surface of Ramos cells is shown in FIG. 1, and the binding of the chimeric antibodies to CD79b on the surface of BJAB cells is shown in FIG. 2.

### Example 5. Humanization of Chimeric Antibodies

The chimeric antibodies obtained in Example 2 were humanized according to a conventional method. Thus, humanized antibodies hz34F1.14, hz11G10.9.p1, hz38D9B3.11, hz54H6A3.13, and hz60D6H6.2, and their CDR sequences, light and heavy chain variable region sequences, and light and heavy chain amino acid sequences are shown in Example 9.

### Example 6. Binding Kinetics of Humanized Antibodies for Antigens as Determined by ForteBio

The equilibrium dissociation constants (KD) for the binding of the humanized antibodies of the present invention to human CD79b were determined by ForteBio. The assay was the same as in Example 3, and the data are shown in Table 3.

**Table 3. Affinity constants (M) for binding of antigens and antibodies by ForteBio assay**

| **Sample ID** | KD (M) | | |
|---|---|---|---|
| | **huCD79b** | **huCD79b-N** | **huCD79b-C** |
| hz34F1.14 | 2.53E-09 | 8.07E-09 | NB |
| hz11G10.9.p1 | 1.35E-09 | 3.40E-09 | NB |
| hz38D9B3.11 | 2.74E-10 | 2.75E-10 | NB |
| hz54H6A3.13 | 3.08E-09 | 6.88E-09 | NB |
| hz60D9H6.2 | 3.39E-09 | 2.18E-08 | NB |
| A7v14b | 1.84E-09 | 8.56E-09 | NB |
| polmab | 1.27E-08 | 3.22E-09 | NB |

As can be seen from the affinity of the humanized antibodies for CD79b listed in Table 3, the humanized antibodies substantially maintained the binding of the previous chimeric antibodies to CD79b.

### Example 7. Binding Assays for Humanized Antibodies and B-Cell Non-Hodgkin's Lymphoma Cell Lines

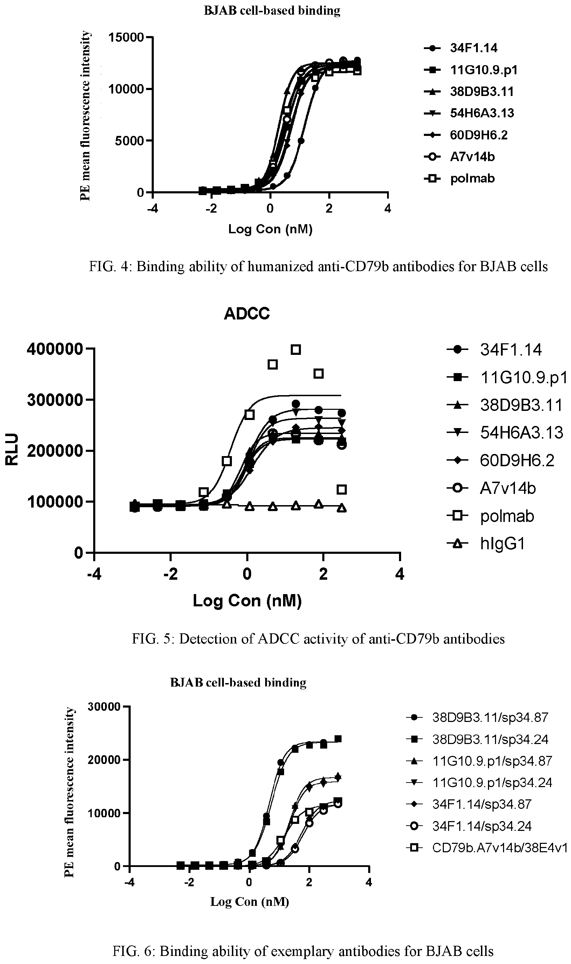

The binding of the humanized antibodies to Ramos and BJAB cells was detected by flow cytometry. The assay was the same as in Example 4. The binding of the humanized antibodies to CD79b on the surface of Ramos cells is shown in FIG. 3; the binding of the humanized antibodies to CD79b on the surface of BJAB cells is shown in FIG. 4.

### Example 8. Effect of Antibody-Mediated ADCC Effect

This example investigates the effect of the obtained antibodies in mediating an ADCC effect and thereby eliminating tumor cells. In this study, the Jurkat-ADCC NF-AT luciferase effector cell strain (hereinafter referred to as ADCC effector cells) from Promega was used. The ADCC activity of the antibodies was detected by detecting the activation of NF-AT signal. The specific experimental process is as follows:
1) Preparation of cells
   The target cells 293T-hCD79b and ADCC effector cells were counted. The supernatant was removed by centrifugation. The cells were washed twice with PBS and resuspended in a detection medium (1640 medium with 5% low IgG serum (Gibco)). The concentration of ADCC effector cells was adjusted to 2 × 10⁷ cells/mL and the concentration of the target cells was adjusted to 2 × 10⁶ cells/mL. The two types of cells were mixed at a ratio of 1:1, and the final effector/target ratio was 10:1.
2) Plating: the mixed cells were plated at 50 µL/well.
3) Antibody dilution: the antibody samples were each diluted in gradient. The final concentration was 300 nM in the first well, and 4-fold dilution was performed to obtain 10 gradients. 50 µL of antibody was added to the 50 µL of cells in each well.
4) The above 96-well plate was incubated in an incubator at 37 °C for 7 h.
5) After 7 h, the 96-well plate was taken out and the thawed Luciferase assay reagent was added at 100 µL/well. The cells were incubated at room temperature for 20 min and detected using a microplate reader. The concentration-dependent curve was fitted with GraphPad.

As shown in FIG. 5, the humanized antibodies can effectively activate the NF-AT signaling pathway downstream of ADCC.

### Example 9. Expression and Purification of Bispecific Antibodies of the Present Invention

According to the combinations shown in Table 4, 3 different anti-human CD79b clones 38D9B3.11, 11G10.9.p1, and 34F1.14 were each combined with 2 different anti-human CD3 clones HzSP34.87 and HzSP34.24 to separately construct a total of 6 bispecific antibodies 38D9B3.11/sp34.87, 38D9B3.11/sp34.24, 11G10.9.p1/sp34.87, 11G10.9.p1/sp34.24, 34F1.14/sp34.87, and 34F1.14/sp34.24. The bispecific antibodies were each transiently expressed in HEK293 cells. After 7 days of expression, the resulting cell fermentation broth was filtered and clarified, and then a Protein A column (Hitrap Mabselect Sure, GE 11-0034-95) was used for trapping to obtain half antibodies. After the concentration of the half antibodies was detected by the A280 method, the half antibodies were mixed in a molar ratio of 1:1. A proper amount of the reducing agent GSH was added, and the mixture was reacted at room temperature overnight. Then the reducing agent was removed by ultrafiltration, and the reaction was terminated. Fine purification was then performed using MonoS cation exchange chromatography. The eluate was ultrafiltered and buffer exchanged into PBS (Gibco, 70011-044); the molecular weight was determined by mass spectrometry and the purity was determined by SEC-HPLC. CD79b.A7v14b/38E4v1 is a CD79b/CD3 bispecific antibody from patent US20180327492 and was expressed using the same method. The above 7 bispecific antibodies obtained were used in the following examples.

**Table 4. List of CD79b/CD3 bispecific antibodies**

| | Anti-CD79b end | Anti-CD3 end |
|---|---|---|
| 1 | 38D9B3.11 | HzSP34.87 |
| 2 | 38D9B3.11 | HzSP34.24 |
| 3 | 11G10.9.p1 | HzSP34.87 |
| 4 | 11G10.9.p1 | HzSP34.24 |
| 5 | 34F1.14 | HzSP34.87 |
| 6 | 34F1.14 | HzSP34.24 |
| 7 | CD79b.A7v14b | 38E4v1 |

The specific amino acid sequence listing (CDR regions defined by Kabat rules) is as follows:

### Heavy and light chain amino acid sequences of38D9B3.11

Heavy chain CDR sequences are as follows:
   HCDR1 (SEQ ID NO: 1): GYTFTDYSMH;
   HCDR2 (SEQ ID NO: 2): WINTETGEPSYADDFKG;
   HCDR3 (SEQ ID NO: 3): GPY;
Heavy chain variable region VH sequence (SEQ ID NO: 4): 
QVQLVQSGSELKKPGASVKVSCKASGYTFTDYSMHWVRQAPGQGLEWMGWINTETGEPSYADDFKGR
FVFSLDTSVSTAYLQISSLKAEDTAVYYCYYGPYWGQGTLVTVSS;
Full-length heavy chain sequence (SEQ ID NO: 5): 
QVQLVQSGSELKKPGASVKVSCKASGYTFTDYSMHWVRQAPGQGLEWMGWINTETGEPSYADDFKGR
FVFSLDTSVSTAYLQISSLKAEDTAVYYCYYGPYWGQGTLVTVSSASTKGPSVFPLAPSSKSTSGGTAALG
CLVKDYFPEPVTVSWNSGALTSGVHTFPAVLQSSGLYSLSSVVTVPSSSLGTQTYICNVNHKPSNTKVDKK
VEPKSCDKTHTCPPCPAPEAAGGPSVFLFPPKPKDTLMISRTPEVTCVVVDVSHEDPEVKFNWYVDGVEV
HNAKTKPREEQYNSTYRVVSVLTVLHQDWLNGKEYKCKVSNKALPAPIEKTISKAKGQPREPQVYTLPPS
RDELTKNQVSLWCLVKGFYPSDIAVEWESNGQPENNYKTTPPVLDSDGSFFLYSKLTVDKSRWQQGNVFS
CSVMHEALHNHYTQKSLSLSPGK.
Light chain CDR sequences:
   LCDR1 (SEQ ID NO:6): KASQSVDHDVDSYMD;
   LCDR2 (SEQ ID NO:7): SASNLES;
   LCDR3 (SEQ ID NO:8): QQINEYPYT;
Light chain variable region VL sequence (SEQ ID NO: 9): 
EIVLTQSPATLSLSPGERATLSCKASQSVDHDVDSYMDWYQQKPGQAPRLLIYSASNLESGIPARFSGSGS
GTDFTLTISSLEPEDFAVYYCQQINEYPYTFGGGTKVEIK;
Full-length light chain sequence (SEQ ID NO: 10): 
EIVLTQSPATLSLSPGERATLSCKASQSVDHDVDSYMDWYQQKPGQAPRLLIYSASNLESGIPARFSGSGS
GTDFTLTISSLEPEDFAVYYCQQINEYPYTFGGGTKVEIKRTVAAPSVFIFPPSDEQLKSGTASVVCLLNNFY
 
PREAKVQWKVDNALQSGNSQESVTEQDSKDSTYSLSSTLTLSKADYEKHKVYACEVTHQGLSSPVTKSF
NRGEC.
Heavy and light chain amino acid sequences of 11G10.9.p1
Heavy chain CDR sequences are as follows:
   HCDR1 (SEQ ID NO:11): GYTFTDYWVN;
   HCDR2 (SEQ ID NO:12): MIDPSDSETHYNQMFND;
   HCDR3 (SEQ ID NO:13): SNYY;
Heavy chain variable region VH sequence (SEQ ID NO: 14): 
QVQLVQSGAEVKKPGASVKVSCKASGYTFTDYWVNWVRQAPGQGLEWMGMIDPSDSETHYNQMFND
RVTMTVDTSTSTVYMELSSLRSEDTAVYYCTRSNYYWGQGTLVTVSS;
Full-length heavy chain sequence (SEQ ID NO: 15): 
QVQLVQSGAEVKKPGASVKVSCKASGYTFTDYWVNWVRQAPGQGLEWMGMIDPSDSETHYNQMFND
RVTMTVDTSTSTVYMELSSLRSEDTAVYYCTRSNYYWGQGTLVTVSSASTKGPSVFPLAPSSKSTSGGTA
ALGCLVKDYFPEPVTVSWNSGALTSGVHTFPAVLQSSGLYSLSSVVTVPSSSLGTQTYICNVNHKPSNTKV
DKKVEPKSCDKTHTCPPCPAPEAAGGPSVFLFPPKPKDTLMISRTPEVTCVVVDVSHEDPEVKFNWYVDG
VEVHNAKTKPREEQYNSTYRVVSVLTVLHQDWLNGKEYKCKVSNKALPAPIEKTISKAKGQPREPQVYT
LPPSRDELTKNQVSLWCLVKGFYPSDIAVEWESNGQPENNYKTTPPVLDSDGSFFLYSKLTVDKSRWQQG
NVFSCSVMHEALHNHYTQKSLSLSPGK.
The light chain CDR sequences are as follows:
   LCDR1 (SEQ ID NO:16): KSSQSLLDSEGKTYLN;
   LCDR2 (SEQ ID NO: 17): LVSKLDS;
   LCDR3 (SEQ ID NO:18): GTHFPLT;
Light chain variable region VL sequence (SEQ ID NO: 19): 
DIVMTQTPLSLSVTPGQPASISCKSSQSLLDSEGKTYLNWLLQKPGQSPQSLIYLVSKLDSGVPDRFSGSGS
GTDFTLKISRVEAEDVGVYYCWQGTHFPLTFGGGTKVEIK;
Full-length light chain sequence (SEQ ID NO: 20): 
DIVMTQTPLSLSVTPGQPASISCKSSQSLLDSEGKTYLNWLLQKPGQSPQSLIYLVSKLDSGVPDRFSGSGS
GTDFTLKISRVEAEDVGVYYCWQGTHFPLTFGGGTKVEIKRTVAAPSVFIFPPSDEQLKSGTASVVCLLNN
FYPREAKVQWKVDNALQSGNSQESVTEQDSKDSTYSLSSTLTLSKADYEKHKVYACEVTHQGLSSPVTK
SFNRGEC.
Heavy and light chain amino acid sequences of 34F1.14
Heavy chain CDR sequences are as follows:
   HCDR1 (SEQ ID NO:21): GYSFTTYWMN;
   HCDR2 (SEQ ID NO:22): MIDPSDSETHYNHLFKD;
   HCDR3 (SEQ ID NO:23): AIGY;
Heavy chain variable region VH sequence (SEQ ID NO: 24): 
QVQLVQSGAEVKKPGASVKVSCKASGYSFTTYWMNWVRQAPGQGLTWMGMIDPSDSETHYNHLFKDR
VTMTVDKSTSTAYMELSSLRSEDTAVYYCTRAIGYWGQGTLVTVSS;
Full-length heavy chain sequence (SEQ ID NO: 25): 
QVQLVQSGAEVKKPGASVKVSCKASGYSFTTYWMNWVRQAPGQGLTWMGMIDPSDSETHYNHLFKDR
VTMTVDKSTSTAYMELSSLRSEDTAVYYCTRAIGYWGQGTLVTVSSASTKGPSVFPLAPSSKSTSGGTAA
LGCLVKDYFPEPVTVSWNSGALTSGVHTFPAVLQSSGLYSLSSVVTVPSSSLGTQTYICNVNHKPSNTKVD
KKVEPKSCDKTHTCPPCPAPEAAGGPSVFLFPPKPKDTLMISRTPEVTCVVVDVSHEDPEVKFNWYVDGV
EVHNAKTKPREEQYNSTYRVVSVLTVLHQDWLNGKEYKCKVSNKALPAPIEKTISKAKGQPREPQVYTL
PPSRDELTKNQVSLWCLVKGFYPSDIAVEWESNGQPENNYKTTPPVLDSDGSFFLYSKLTVDKSRWQQGN
VFSCSVMHEALHNHYTQKSLSLSPGK.
The light chain CDR sequences are as follows:
   LCDR1 (SEQ ID NO:26): KSSLSLLDSEGKTYLN;
   LCDR2 (SEQ ID NO:27): LVSKLDS;
   LCDR3 (SEQ ID NO:28): WQGTHFPLT;
Light chain variable region VL sequence (SEQ ID NO: 29): 
DVVMTQSPLSLPVTLGQPASISCKSSLSLLDSEGKTYLNWFQQRPGQSPRPLISLVSKLDSGVPDRFSGSGS
GTDFTLKISRVEAEDVGVYYCWQGTHFPLTFGGGTKVEIK;
Full-length light chain sequence (SEQ ID NO: 30): 
DVVMTQSPLSLPVTLGQPASISCKSSLSLLDSEGKTYLNWFQQRPGQSPRPLISLVSKLDSGVPDRFSGSGS
GTDFTLKISRVEAEDVGVYYCWQGTHFPLTFGGGTKVEIKRTVAAPSVFIFPPSDEQLKSGTASVVCLLNN
FYPREAKVQWKVDNALQSGNSQESVTEQDSKDSTYSLSSTLTLSKADYEKHKVYACEVTHQGLSSPVTK
SFNRGEC.
Heavy and light chain amino acid sequences of HzSP34.87
Heavy chain CDR sequences are as follows:
   HCDR1 (SEQ ID NO:31): GFTFNTYAMN;
   HCDR2 (SEQ ID NO:32): RIRSKYNNYATYYAD;
   HCDR3 (SEQ ID NO:33): HYNFGQSYVSWFAY;
Heavy chain variable region VH sequence (SEQ ID NO: 34): 
EVQLVESGGGLVQPGGSLKLSCAASGFTFNTYAMNWVRQASGKGLEWVGRIRSKYNNYATYYADSVKD
RFTISRDDSKSTLYLQMNSLKTEDTAVYYCARHYNFGQSYVSWFAYWGQGTTVTVSS;
Full-length heavy chain sequence (SEQ ID NO: 35): 
EVQLVESGGGLVQPGGSLKLSCAASGFTFNTYAMNWVRQASGKGLEWVGRIRSKYNNYATYYADSVKD
RFTISRDDSKSTLYLQMNSLKTEDTAVYYCARHYNFGQSYVSWFAYWGQGTTVTVSSASTKGPSVFPLAP
SSKSTSGGTAALGCLVKDYFPEPVTVSWNSGALTSGVHTFPAVLQSSGLYSLSSVVTVPSSSLGTQTYICNV
NHKPSNTKVDKKVEPKSCDKTHTCPPCPAPEAAGGPSVFLFPPKPKDTLMISRTPEVTCVVVDVSHEDPE
VKFNWYVDGVEVHNAKTKPREEQYNSTYRVVSVLTVLHQDWLNGKEYKCKVSNKALPAPIEKTISKAK
GQPREPQVYTLPPSRDELTKNQVSLSCAVKGFYPSDIAVEWESNGQPENNYKTTPPVLDSDGSFFLVSKLT
VDKSRWQQGNVFSCSVMHEALHNHYTQKSLSLSPGK.
The light chain CDR sequences are as follows:
   LCDR1 (SEQ ID NO:36): RSSTGAVTTSNYAN;
   LCDR2 (SEQ ID NO:37): GTNKRAP;
   LCDR3 (SEQ ID NO:38): ALWYSNLWV;
Light chain variable region VL sequence (SEQ ID NO: 39): 
QAVVTQEPSLTVSPGGTVTLTCRSSTGAVTTSNYANWVQQKPGQAPRGLIGGTNKRAPGVPARFSGSLLG
DKAALTLLGAQPEDEAEYYCALWYSNLWVFGQGTKLTVL;
Full-length light chain sequence (SEQ ID NO: 40): 
QAVVTQEPSLTVSPGGTVTLTCRSSTGAVTTSNYANWVQQKPGQAPRGLIGGTNKRAPGVPARFSGSLLG
DKAALTLLGAQPEDEAEYYCALWYSNLWVFGQGTKLTVLGQPKAAPSVTLFPPSSEELQANKATLVCLIS
DFYPGAVTVAWKADSSPVKAGVETTTPSKQSNNKYAASSYLSLTPEQWKSHRSYSCQVTHEGSTVEKTV
APTECS.
Heavy and light chain amino acid sequences of HzSP34.24
Heavy chain CDR sequences are as follows:
   HCDR1 (SEQ ID NO:41): GFTFNTYAMN;
   HCDR2 (SEQ ID NO:42): RIRSKYNNYATYYADSVKD;
   HCDR3 (SEQ ID NO:43): HGNFGQSYVSWFAY;
Heavy chain variable region VH sequence (SEQ ID NO: 44): 
EVQLVESGGGLVQPGGSLKLSCAASGFTFNTYAMNWVRQASGKGLEWVGRIRSKYNNYATYYADSVKD
RFTISRDDSKSTLYLQMNSLKTEDTAVYYCARHGNFGQSYVSWFAYWGQGTTVTVSS;
Full-length heavy chain sequence (SEQ ID NO: 45): 
EVQLVESGGGLVQPGGSLKLSCAASGFTFNTYAMNWVRQASGKGLEWVGRIRSKYNNYATYYADSVKD
RFTISRDDSKSTLYLQMNSLKTEDTAVYYCARHGNFGQSYVSWFAYWGQGTTVTVSSASTKGPSVFPLAP
SSKSTSGGTAALGCLVKDYFPEPVTVSWNSGALTSGVHTFPAVLQSSGLYSLSSVVTVPSSSLGTQTYICNV
NHKPSNTKVDKKVEPKSCDKTHTCPPCPAPEAAGGPSVFLFPPKPKDTLMISRTPEVTCVVVDVSHEDPE
VKFNWYVDGVEVHNAKTKPREEQYNSTYRVVSVLTVLHQDWLNGKEYKCKVSNKALPAPIEKTISKAK
GQPREPQVYTLPPSRDELTKNQVSLSCAVKGFYPSDIAVEWESNGQPENNYKTTPPVLDSDGSFFLVSKLT
VDKSRWQQGNVFSCSVMHEALHNHYTQKSLSLSPGK.
The light chain CDR sequences are as follows:
   LCDR1 (SEQ ID NO:46): RSSTGAVTTSNYAN;
   LCDR2 (SEQ ID NO:47): GTNKRAP;
   LCDR3 (SEQ ID NO:48): ALWYSNLWV;
Light chain variable region VL sequence (SEQ ID NO: 49): 
QAVVTQEPSLTVSPGGTVTLTCRSSTGAVTTSNYANWVQQKPGQAPRGLIGGTNKRAPGVPARFSGSLLG
DKAALTLLGAQPEDEAEYYCALWYSNLWVFGQGTKLTVL;
Full-length light chain sequence (SEQ ID NO: 50):

QAVVTQEPSLTVSPGGTVTLTCRSSTGAVTTSNYANWVQQKPGQAPRGLIGGTNKRAPGVPARFSGSLLG
DKAALTLLGAQPEDEAEYYCALWYSNLWVFGQGTKLTVLGQPKAAPSVTLFPPSSEELQANKATLVCLIS
DFYPGAVTVAWKADSSPVKAGVETTTPSKQSNNKYAASSYLSLTPEQWKSHRSYSCQVTHEGSTVEKTV
APTECS.

### Heavy and light chain amino acid sequences of CD79b.A7v14b

Heavy chain CDR sequences are as follows:
   HCDR1 (SEQ ID NO:51): GYTFTTYYMN;
   HCDR2 (SEQ ID NO:52): MIDPSDSETHYNQKFQG;
   HCDR3 (SEQ ID NO:53): SLAF;
Heavy chain variable region VH sequence (SEQ ID NO: 54): 
EVQLVQSGAEVKKPGASVKVSCKASGYTFTTYYMNWVRQAPGQGLEWIGMIDPSDSETHYNQKFQGR
ATLTVDTSTSTAYLELSSLRSEDTAVYYCSRSLAFWGQGTLVTVSS;
Full-length heavy chain sequence (SEQ ID NO: 55): 
EVQLVQSGAEVKKPGASVKVSCKASGYTFTTYYMNWVRQAPGQGLEWIGMIDPSDSETHYNQKFQGR
ATLTVDTSTSTAYLELSSLRSEDTAVYYCSRSLAFWGQGTLVTVSSASTKGPSVFPLAPSSKSTSGGTAALG
CLVKDYFPEPVTVSWNSGALTSGVHTFPAVLQSSGLYSLSSVVTVPSSSLGTQTYICNVNHKPSNTKVDKK
VEPKSCDKTHTCPPCPAPEAAGGPSVFLFPPKPKDTLMISRTPEVTCVVVDVSHEDPEVKFNWYVDGVEV
HNAKTKPREEQYNSTYRVVSVLTVLHQDWLNGKEYKCKVSNKALPAPIEKTISKAKGQPREPQVYTLPPS
RDELTKNQVSLWCLVKGFYPSDIAVEWESNGQPENNYKTTPPVLDSDGSFFLYSKLTVDKSRWQQGNVFS
CSVMHEALHNHYTQKSLSLSPGK.
The light chain CDR sequences are as follows:
   LCDR1 (SEQ ID NO:56): KSSQSLLDSDGKTYLN;
   LCDR2 (SEQ ID NO:57): LVSKLDS;
   LCDR3 (SEQ ID NO:58): WQGTHFPQT;
Light chain variable region VL sequence (SEQ ID NO: 59): 
DIVMTQTPLSLPVTPGQPASISCKSSQSLLDSDGKTYLNWLLQKPGQSPQSLIYLVSKLDSGVPDRFSGSGS
GTDFTLKISRVEAEDVGVYYCWQGTHFPQTFGQGTKVEIK;
Full-length light chain sequence (SEQ ID NO: 60):

DIVMTQTPLSLPVTPGQPASISCKSSQSLLDSDGKTYLNWLLQKPGQSPQSLIYLVSKLDSGVPDRFSGSGS
GTDFTLKISRVEAEDVGVYYCWQGTHFPQTFGQGTKVEIKRTVAAPSVFIFPPSDEQLKSGTASVVCLLNN
FYPREAKVQWKVDNALQSGNSQESVTEQDSKDSTYSLSSTLTLSKADYEKHKVYACEVTHQGLSSPVTK
SFNRGEC.

### Heavy and light chain amino acid sequences of 38E4v1

Heavy chain CDR sequences are as follows:
   HCDR1 (SEQ ID NO:61): GFTFTSYYIH;
   HCDR2 (SEQ ID NO:62): WIYPENDNTKYNEKFKD;
   HCDR3 (SEQ ID NO:63): DGYSRYYFDY;
Heavy chain variable region VH sequence (SEQ ID NO: 64): 
EVQLVQSGAEVKKPGASVKVSCKASGFTFTSYYIHWVRQAPGQGLEWIGWIYPENDNTKYNEKFKDRV
TITADTSTSTAYLELSSLRSEDTAVYYCARDGYSRYYFDYWGQGTLVTVSS;
Full-length heavy chain sequence (SEQ ID NO: 65): 
EVQLVQSGAEVKKPGASVKVSCKASGFTFTSYYIHWVRQAPGQGLEWIGWIYPENDNTKYNEKFKDRV
TITADTSTSTAYLELSSLRSEDTAVYYCARDGYSRYYFDYWGQGTLVTVSSASTKGPSVFPLAPSSKSTSG
 
GTAALGCLVKDYFPEPVTVSWNSGALTSGVHTFPAVLQSSGLYSLSSVVTVPSSSLGTQTYICNVNHKPSN
TKVDKKVEPKSCDKTHTCPPCPAPEAAGGPSVFLFPPKPKDTLMISRTPEVTCVVVDVSHEDPEVKFNWY
VDGVEVHNAKTKPREEQYNSTYRVVSVLTVLHQDWLNGKEYKCKVSNKALPAPIEKTISKAKGQPREPQ
VYTLPPSRDELTKNQVSLSCAVKGFYPSDIAVEWESNGQPENNYKTTPPVLDSDGSFFLVSKLTVDKSRW
QQGNVFSCSVMHEALHNHYTQKSLSLSPGK.
The light chain CDR sequences are as follows:
   LCDR1 (SEQ ID NO:66): KSSQSLLNSRTRKNYLA;
   LCDR2 (SEQ ID NO:67): WTSTRKS;
   LCDR3 (SEQ ID NO:68): KQSFILRT;
Light chain variable region VL sequence (SEQ ID NO: 69): 
DIVMTQSPDSLAVSLGERATINCKSSQSLLNSRTRKNYLAWYQQKPGQSPKLLIYWTSTRKSGVPDRFSGS
GSGTDFTLTISSLQAEDVAVYYCKQSFILRTFGQGTKVEIK;
Full-length light chain sequence (SEQ ID NO: 70):

DIVMTQSPDSLAVSLGERATINCKSSQSLLNSRTRKNYLAWYQQKPGQSPKLLIYWTSTRKSGVPDRFSGS
GSGTDFTLTISSLQAEDVAVYYCKQSFILRTFGQGTKVEIKRTVAAPSVFIFPPSDEQLKSGTASVVCLLNNF
YPREAKVQWKVDNALQSGNSQESVTEQDSKDSTYSLSSTLTLSKADYEKHKVYACEVTHQGLSSPVTKS
FNRGEC.

### Heavy and light chain amino acid sequences of polmab:

Full-length heavy chain sequence (SEQ ID NO: 71): 
EVQLVESGGGLVQPGGSLRLSCAASGYTFSSYWIEWVRQAPGKGLEWIGEILPGGGDTNYNEIFKGRATF
SADTSKNTAYLQMNSLRAEDTAVYYCTRRVPIRLDYWGQGTLVTVSSASTKGPSVFPLAPSSKSTSGGTA
ALGCLVKDYFPEPVTVSWNSGALTSGVHTFPAVLQSSGLYSLSSVVTVPSSSLGTQTYICNVNHKPSNTKV
DKKVEPKSCDKTHTCPPCPAPELLGGPSVFLFPPKPKDTLMISRTPEVTCVVVDVSHEDPEVKFNWYVDG
VEVHNAKTKPREEQYNSTYRVVSVLTVLHQDWLNGKEYKCKVSNKALPAPIEKTISKAKGQPREPQVYT
LPPSRDELTKNQVSLTCLVKGFYPSDIAVEWESNGQPENNYKTTPPVLDSDGSFFLYSKLTVDKSRWQQG
NVFSCSVMHEALHNHYTQKSLSLSPGK
Full-length light chain sequence (SEQ ID NO: 72):

DIQLTQSPSSLSASVGDRVTITCKASQSVDYEGDSFLNWYQQKPGKAPKLLIYAASNLESGVPSRFSGSGS
GTDFTLTISSLQPEDFATYYCQQSNEDPLTFGQGTKVEIKRTVAAPSVFIFPPSDEQLKSGTASVVCLLNNFY
PREAKVQWKVDNALQSGNSQESVTEQDSKDSTYSLSSTLTLSKADYEKHKVYACEVTHQGLSSPVTKSF
NRGEC

### Example 10. Affinity Assays of Antibodies of the Present Invention

The binding kinetics of exemplary anti-CD79b/CD3 antibodies of the present invention for human CD79b, human CD3, and cynomolgus monkey CD3 were determined using surface plasmon resonance (Biacore T200). (1) Affinity assay of antibodies for human CD79b-his (Sino biological): 50 mM N-hydroxysuccinimide (NHS) and 200 mM 1-ethyl-3-(3-dimethylaminopropyl)carbodiimide hydrochloride (EDC) were freshly mixed and activated for 420 s at a flow rate of 10 µL/min. Human CD79b-His antigen was then diluted in 10 mM Acetate (pH 5.0), and after dilution to a concentration of 1 µg/mL, the antigen solution was subjected to coupling and the value was approximately 50 RU. Then 1 M ethanolamine was injected at a flow rate of 10 µL/min for 420 s to block the remaining activation sites. The buffer used in the experiment was HBS-EP+ solution at pH 7.4, and under a high performance mode, the antibodies obtained after 2-fold gradient dilution (0-80 nM) were each injected, according to an ascending order based on concentrations, into the channels 1, 2, 3 and 4 of the chip at a flow rate of 30 µL/min, wherein one cycle was used for determination of one concentration, the binding time was 180 s, and the dissociation time was 600 s. (2) Affinity assay of antibodies for human CD3E & CD3G-biotin (Sino biological) and monkey CD3E & CD3G-biotin (Sino biological): 50 mM NHS and 200 mM EDC were freshly mixed and activated for 420 s at a flow rate of 10 µL/min. Streptavidin (Thermo Fisher scientific) protein was then diluted in 10 mM Acetate (pH 4.5) and after dilution to a concentration of 10 µg/mL, the antigen solution was subjected to coupling and the value was approximately 3000 RU. Then 1 M ethanolamine was injected at a flow rate of 10 µL/min for 420 s to block the remaining activation sites. Human CD3E & CD3G-biotin and monkey CD3E & CD3G-biotin proteins were each immobilized on the chip channels coupled with Streptavidin protein. The antibodies (0-16 nM or 0-40 nM or 0-200 nM) obtained after 2-fold gradient dilution were each injected, according to an ascending order based on concentrations, into the channels 1, 2, 3 and 4 of the chip at a flow rate of 30 µL/min, wherein one cycle was used for determination of one concentration, the binding time was 180 s, and the dissociation time was 300 s.

In experiments performed using the assay described above, the affinities of 38D9B3.11/sp34.87, 38D9B3.11/sp34.24, 11G10.9.p1/sp34.87, 11G10.9.p1/sp34.24, 34F1.14/sp34.87, 34F1.14/sp34.24, and CD79b.A7v14b/38E4v1 for human CD79b-His are shown in Table 5. The affinities of 38D9B3.11/sp34.87, 38D9B3.11/sp34.24, 11G10.9.p1/sp34.87, 11G10.9.p1/sp34.24, 34F1.14/sp34.87, 34F1.14/sp34.24, and CD79b.A7v14b/38E4v1 for human CD3E & CD3G (Sino biological) and monkey CD3E & CD3G are shown in Tables 6 and 7.

**Table 5. Detection of affinities of exemplary antibodies for human CD79b**

| Name | Antigen | Ka (1/Ms) | Kd (1/s) | KD (M) |
|---|---|---|---|---|
| 38D9B3.11/sp34.87 | Human CD79b-His | 1.87E+05 | 3.10E-04 | 1.66E-09 |
| 38D9B3.11/sp34.24 | | 3.41E+05 | 2.68E-04 | 7.87E-10 |
| 11G10.9.p1/sp34.87 | | 4.61E+05 | 2.08E-03 | 4.50E-09 |
| 11G10.9.p1/sp34.24 | | 8.15E+05 | 1.76E-03 | 2.16E-09 |
| 34F1.14/sp34.87 | | 2.95E+05 | 2.07E-03 | 7.03E-09 |
| 34F1.14/sp34.24 | | 5.19E+05 | 1.61E-03 | 3.10E-09 |
| CD79b.A7v14b/38E4v1 | | 8.09E+05 | 7.01E-03 | 8.66E-09 |

**Table 6. Detection of affinities of exemplary antibodies for human CD3E&G**

| Name | Antigen | Ka (1/Ms) | Kd (1/s) | KD (M) |
|---|---|---|---|---|
| 38D9B3.11/sp34.87 | Human CD3E&G | 3.10E+05 | 3.68E-02 | 1.19E-07 |
| 38D9B3.11/sp34.24 | | 9.12E+05 | 5.65E-03 | 6.19E-09 |
| 11G10.9.p1/sp34.87 | | 3.08E+05 | 3.31E-02 | 1.07E-07 |
| 11G10.9.p1/sp34.24 | | 8.51E+05 | 5.31E-03 | 6.25E-09 |
| 34F1.14/sp34.87 | | 3.42E+05 | 3.15E-02 | 9.20E-08 |
| 34F1.14/sp34.24 | | 1.03E+06 | 5.12E-03 | 4.99E-09 |
| CD79b.A7v14b/38E4v1 | | 2.96E+06 | 2.96E-03 | 1.00E-09 |

**Table 7. Detection of affinities of exemplary antibodies for monkey CD3E&G**

| Name | Antigen | Ka (1/Ms) | Kd (1/s) | KD (M) |
|---|---|---|---|---|
| 38D9B3.11/sp34.87 | Cyno CD3E&G | 1.09E+05 | 2.75E-02 | 2.53E-07 |
| 38D9B3.11/sp34.24 | | 6.03E+05 | 6.56E-03 | 1.09E-08 |
| 11G10.9.p1/sp34.87 | | 1.67E+05 | 3.36E-02 | 2.02E-07 |
| 11G10.9.p1/sp34.24 | | 5.70E+05 | 6.10E-03 | 1.07E-08 |
| 34F1.14/sp34.87 | | 1.78E+05 | 3.20E-02 | 1.80E-07 |
| 34F1.14/sp34.24 | | 6.78E+05 | 5.93E-03 | 8.75E-09 |
| CD79b.A7v14b/38E4v1 | | 1.88E+06 | 2.85E-03 | 1.52E-09 |

### Example 11. Binding Assays for the Anti-CD79b/CD3 Antibodies of the Present Invention and B-Cell Non-Hodgkin's Lymphoma Cell Lines

The binding ability of the CD79b/CD3 bispecific antibodies for CD79b-positive B-cell non-Hodgkin's lymphoma cell lines was detected by flow cytometry.

BJAB (AddexBio, USA, Cat# C0003016) and WSU-DLCL2 (Nanjing Kebai Biotechnology Co., Ltd., Cat# CBP60273) were cultured and passaged according to the conventional procedures. Cells were counted after centrifugation and resuspension, adjusted to a density of 4 × 10⁶ cells/mL, poured into a loading tank, and seeded to a 96-well plate at 50 µL/well using a multichannel pipettor. 50 µL of the antibody sample diluted in gradient was added to the 50 µL of cells in each well, and the mixture was then incubated in an incubator at 5% CO₂ and 37 °C for 30 min. The mixture was centrifuged at 400 g for 5 min, then 200 µL of PBS was added, and the resulting mixture was centrifuged at 400 g for 5 min. This procedure was repeated 3 times. Goat anti-human IgG PE (1:200) was added, and the mixture was incubated for 30 min in the dark at room temperature. The mixture was washed with 200 µL of PBS twice, centrifuged, and then resuspended in 100 µL of PBS, and the values were read using a flow cytometer. The values were fitted to obtain a curve and EC₅₀ values were calculated.

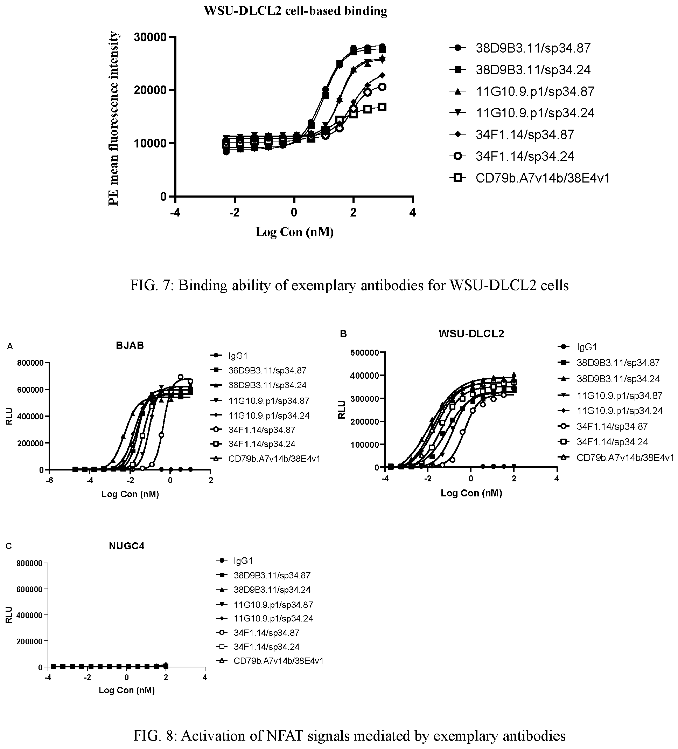

In experiments performed using the assay described above, the binding of the exemplary antibodies to CD79b on the surface of the BJAB cells, a CD79b high expressing cell line, is shown in FIG. 6, and the binding of the exemplary antibodies to CD79b on the surface of the WSU-DLCL2 cells, a CD79b low expressing cell line, is shown in FIG. 7.

### Example 12. Assays on Activation of Jurkat-NFAT-Luc Cells by Anti-CD79b/CD3 Antibodies of the Present Invention

The CD79b/CD3 bispecific antibodies simultaneously bind to CD79b on the surface of B-cell non-Hodgkin's lymphoma cells and CD3 on the surface of Jurkat-NFAT-Luc cells and activate the signaling pathway downstream of NFAT-Luc through CD79b-dependent CD3 cross-linking. In this study, based on the luciferase reporter gene method, the luciferase expression after the co-cultured Jurkat-NFAT-Luc cells and CD79b-positive cells were cultured overnight together with the added exemplary antibody was detected to reflect the activation capacity of the antibody.

### Experimental method

### Preparation of assay medium: 10% FBS + 90% RPMI Medium 1640

Preparation of Bio-Glo Luciferase Assay System detection solution: Bio-Glo^{™} buffer (Promega, G7940) was thawed and mixed well with Bio-GloTM substrate (Promega, G7940), and the mixture was stored in a refrigerator at -40 °C.
1. BJAB, WSU-DLCL2, NUGC4, and Jurkat-NFAT-Luc cells were each centrifuged at 300 g for 8 min, and the supernatant was discarded. The BJAB cells and the Jurkat-NFAT-Luc cells were each suspended in the Assay medium, and then the cells were counted, and the cell density was adjusted, wherein the cell density was 6.0 × 10⁵ for both. The tumor cells and the Jurkat-NFAT-Luc cells were mixed at a ratio of 1:1 for later use.
2. The cell suspension and the test antibody diluted in gradient were added to a 96-well white cell culture plate at 100 µL/well, and the mixture was incubated overnight.
3. The Bio-Glo Luciferase Assay System detection solution was taken out and thawed at room temperature.
4. The sample incubated in step 2 was taken out, and the Bio-Glo Luciferase Assay System detection solution was added at 80 µL/well.
5. The plate was read on a microplate reader (Molecular Device, SpectraMax I3).

In experiments performed using the assay described above, the exemplary antibodies could dose-dependently activate NFAT signals (see FIGs. 8A and 8B), and the exemplary antibodies showed no activation on non-target cells NUGC4 (see FIG. 8C).

### Example 13. Assays on Killing of B-cell Non-Hodgkin's Lymphoma Cell Lines by Anti-CD79b/CD3 Antibodies of the Present Invention

The CD79b/CD3 bispecific antibodies simultaneously bind to CD79b on the surface of B-cell non-Hodgkin's lymphoma cells and CD3 on the surface of primary T cells and activate, through CD79b-dependent CD3 cross linking, the T cells and mediate the killing of CD79b-positive tumor cells by the T cells. In this study, based on the propidium iodide (PI) staining method, the PI positive rate of tumor cells 48 h after the exemplary antibody was added to the co-cultured human CD8+ T cells and CD79b-positive cells was detected to evaluate the killing capacity of the human CD8+ T cells on the CD79b-positive tumor cells.

PBMCs were taken out from a liquid nitrogen tank, rapidly thawed at 37 °C, and added dropwise to a preheated 1640 medium containing 10% FBS (containing 0.1% DNase) to obtain 10 mL of a mixed solution. The mixed solution was centrifuged at 400 g for 5 min and resuspended in 10 mL of a 1640 medium containing 10% FBS, and 10 µL of DNase was added. The mixture was left to stand at 37 °C and 5% CO₂ for 2 h. Human CD8+ T cells were isolated using the EasySep^{™} human CD8+ T cell enrichment kit and the operation was carried out as per the instructions. The density of the human CD8+ T cells was adjusted to 1 × 10⁶ cells/mL using a 1640 medium containing 10% FBS and the cells were used as effector cells. CD79b-positive cells BJAB and WSU-DLCL2 were used as target cells, and NUGC4 was used as non-target cells. The cells were centrifuged at 400 g for 5 min, and resuspended in a 1640 medium containing 10% FBS, and CellTrace Far Red Cell staining solution (THERMO FISHER, C34564) was added. The mixture was incubated for 30 min, centrifuged at 400 g for 5 min, and resuspended in a 1640 medium containing 10% FBS, and the cell density was adjusted to 2 × 10⁵ cells/mL. The tumor cell suspension and the human CD8+ T cell suspension were well mixed at a ratio of 1:5, and the resulting cell suspension and a test antibody diluted in gradient were added to a (public) 96-well round-bottom culture plate (with a cover) at 200 µL/well. The cells were placed in a carbon dioxide incubator and stimulated at 37 °C for 48 h. The cells were centrifuged at 400 g for 5 min, the supernatant was removed, and PBS (containing 1:1000 diluted propidium iodide solution) was added at 100 µL/well to resuspend the cells. The mixture was left to stand at 4 °C for 10 min, and the values were read using a flow cytometer (BD, FACS CELESTA).

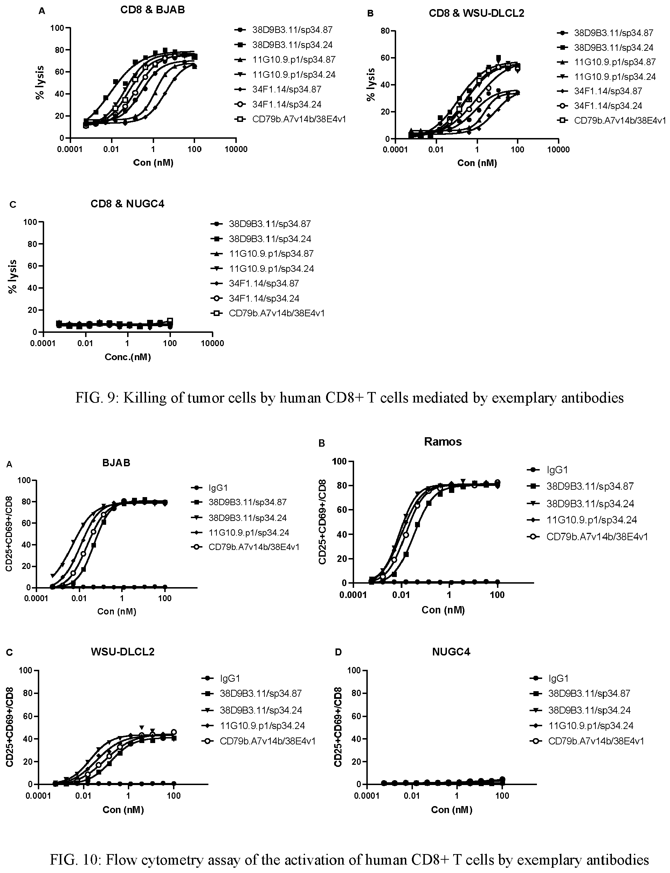

In experiments performed using the assay described above, the exemplary antibodies could dose-dependently induce killing of BJAB and WSU-DLCL2 target cells by human CD8+ T cells (see FIGs. 9A and 9B), and the exemplary antibodies showed no killing against non-target cells NUGC4 (see FIG. 9C).

### Example 14. Levels of T Cell Activation and Cytokine Release during Killing of Human B-Cell Non-Hodgkin's Lymphoma Cell Lines by Anti-CD79b/CD3 Antibodies of the Present Invention

In this study, the exemplary antibody diluted in gradient was added to the co-incubation system of B-cell non-Hodgkin's lymphoma cells and human T-cells, and 18 h later, the percentage of cells double positive for human T-cells CD25 and CD69 was detected by flow cytometry to evaluate the degree of the antibody-mediated activation of T-cells in the co-incubation system of human T-cells and CD79b-positive tumor cells. The levels of various cytokines were simultaneously detected by using a multi-cytokine assay kit (Human Th1/Th2/Th17, BD) to evaluate the antibody-mediated ability of T cells to release cytokines in the co-incubation system of human T-cells and CD79b-positive tumor cells.

### Experimental process

### Detection of human CD8+ T cell activation and cytokine release:

PBMCs were taken out from a liquid nitrogen tank, rapidly thawed at 37 °C, and added dropwise to a preheated 1640 medium containing 10% FBS (containing 0.1% DNase) to obtain 10 mL of a mixed solution. The mixed solution was centrifuged at 400 g for 5 min and resuspended in 10 mL of a 1640 medium containing 10% FBS, and 10 µL of DNase was added. The mixture was left to stand at 37 °C and 5% CO₂ for 2 h. Human CD8+ T cells were isolated using the EasySep^{™} human CD8+ T cell enrichment kit and the operation was carried out as per the instructions. The density of human CD8+ T cells was adjusted to 1 × 10⁶ cells/mL using a 1640 medium containing 10% FBS, and the cells were used as effector cells. Tumor cells were resuspended in a 1640 medium containing 10% FBS, and the density was adjusted to 2 × 10⁵ cells/mL. The tumor cell suspension and the human CD8+ T cell suspension were well mixed at a ratio of 1:1, and the resulting cell suspension and a test antibody diluted in gradient (the highest concentration being 100 nM, 3-fold dilution, 12 gradients) were added to a (public) 96-well round-bottom culture plate (with a cover) at 200 µL/well. The cells were placed in a carbon dioxide incubator and stimulated at 37 °C for 18 h. The cells were centrifuged at 400 g for 5 min, and PBS (containing 1:100 diluted APC anti-human CD8a, FITC anti-human CD69, and PE anti-human CD25) was added at 100 µL/well to resuspend the cells. The mixture was left to stand at 4 °C for 30 min and then washed, and the values were read using a flow cytometer (BD, FACS CELESTA). Meanwhile, the supernatant was collected, and the contents of cytokines TNFα and IFN-γ were detected according to the instructions of the assay kit (Human Th1/Th2/Th17 kit, BD).

### Detection of human CD4+ T cell activation:

PBMCs were taken out from a liquid nitrogen tank, rapidly thawed at 37 °C, and added dropwise to a preheated 1640 medium containing 10% FBS (containing 0.1% DNase) to obtain 10 mL of a mixed solution. The mixed solution was centrifuged at 400 g for 5 min and resuspended in 10 mL of a 1640 medium containing 10% FBS, and 10 µL of DNase was added. The mixture was left to stand at 37 °C and 5% CO₂ for 2 h. Human CD4+ T cells were isolated using the EasySep^{™} human CD4+ T cell enrichment kit and the operation was carried out as per the instructions. The density of human CD4+ T cells was adjusted to 1 × 10⁶ cells/mL using a 1640 medium containing 10% FBS, and the cells were used as effector cells. Tumor cells were resuspended in a 1640 medium containing 10% FBS, and the density was adjusted to 2 × 10⁵ cells/mL. The tumor cell suspension and the human CD4+ T cell suspension were well mixed at a ratio of 1:1, and the resulting cell suspension and a test antibody diluted in gradient were added to a (public) 96-well round-bottom culture plate (with a cover) at 200 µL/well. The cells were placed in a carbon dioxide incubator and stimulated at 37 °C for 18 h. The cells were centrifuged at 400 g for 5 min, the supernatant was removed, and PBS (containing 1:100 diluted APC anti-human CD4, FITC anti-human CD69, and PE anti-human CD25) was added at 100 µL/well to resuspend the cells. The mixture was left to stand at 4 °C for 30 min and then washed, and the values were read using a flow cytometer (BD, FACS CELESTA).

### Results

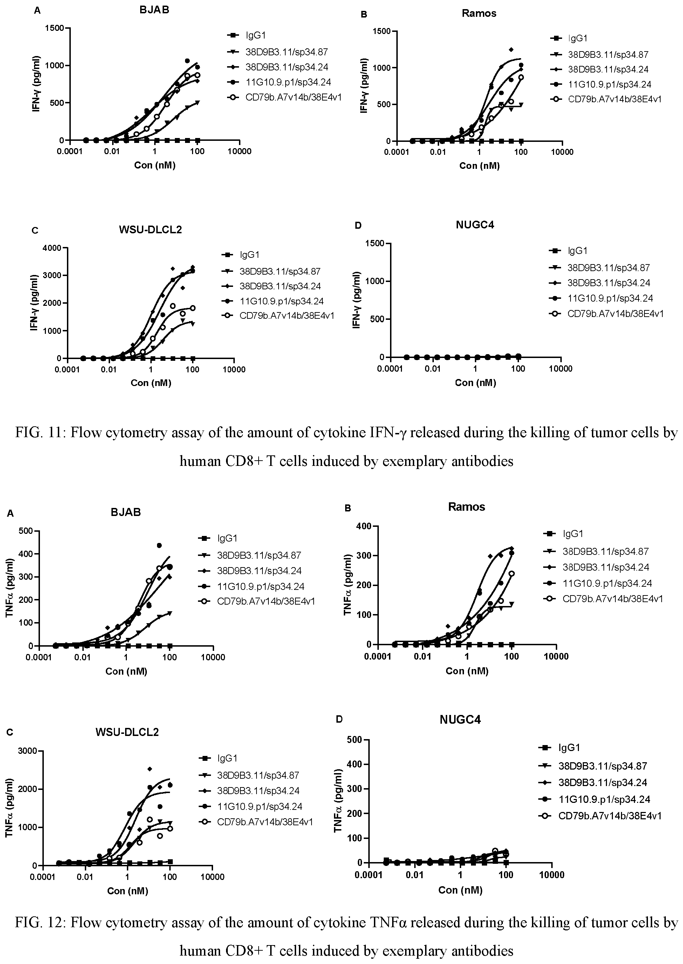

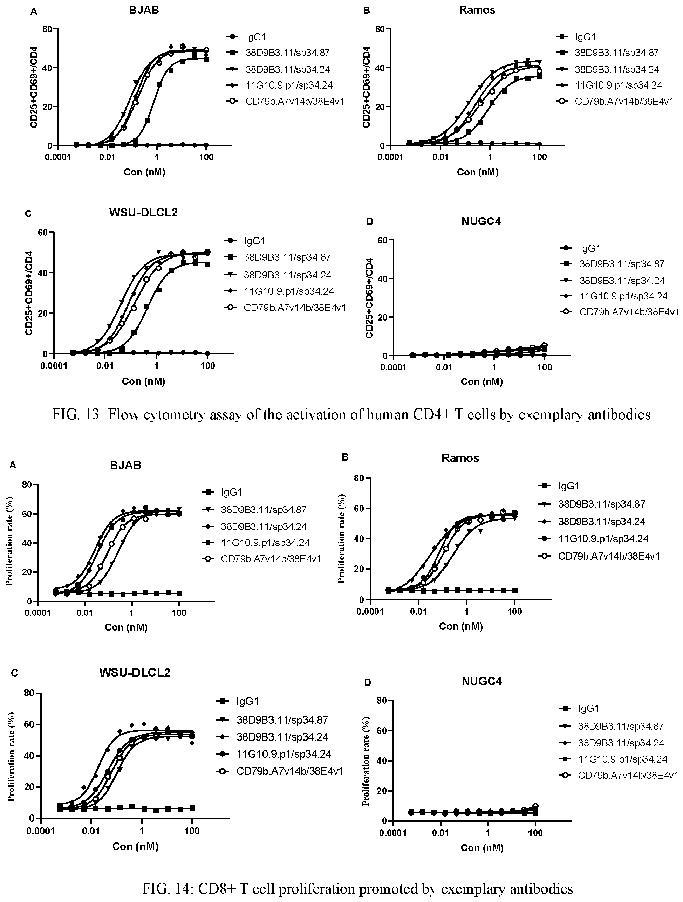

In experiments performed using the assay described above, the degree of CD8+ activation during the exemplary antibody-induced killing of BJAB cells by CD8+ T cells is shown in FIG. 10A; the degree of CD8+ activation during the exemplary antibody-induced killing of Ramos cells by CD8+ T cells is shown in FIG. 10B; the degree of CD8+ activation during the exemplary antibody-induced killing of WSU-DLCL2 cells by CD8+ T cells is shown in FIG. 10C; the degree of CD8+ activation during the exemplary antibody-induced killing of NUGC4 cells by CD8+ T cells is shown in FIG. 10D. The levels of IFN-γ release during the exemplary antibody-induced killing of various cells by CD8+ T cells are shown in FIG. 11; the levels of TNFα release during the exemplary antibody-induced killing of various cells by CD8+ T cells are shown in FIG. 12. The degree of CD4+ activation during the exemplary antibody-induced co-incubation of CD4+ T cells and BJAB cells is shown in FIG. 13A; the degree of CD4+ activation during the exemplary antibody-induced co-incubation of CD4+ T cells and Ramos cells is shown in FIG. 13B; the degree of CD4+ activation during the exemplary antibody-induced co-incubation of CD4+ T cells and WSU-DLCL2 cells is shown in FIG. 13C; the degree of CD4+ activation during the exemplary antibody-induced co-incubation of CD4+ T cells and NUGC4 cells is shown in FIG. 13D. In the presence of CD79b-positive tumor cells, the exemplary antibodies all can dose-dependently activate CD8+ T cells and CD4+ T cells isolated from PBMCs, and the degree of T cell activation correlates somewhat with the affinity for CD3, that is, the higher the affinity for CD3, the greater the ability to activate T cells.

### Example 15. Assays on Promotion of Proliferation Capacity of Human CD8+ T Cells by Exemplary Antibodies

CD8+ T cells isolated from PBMCs were labeled with the CellTrace Far Red Cell Proliferation Kit, and the labeled human CD8+ T cells were co-cultured with tumor cells, followed by addition of the exemplary antibody diluted in gradient, and the proliferation of CD8+ T cells was detected 96 h later by using a flow cytometer.

### Experimental procedures

Isolation of human CD8+ T cells: PBMCs were taken out from a liquid nitrogen tank, rapidly thawed at 37 °C, and added dropwise to 10 mL of 1640 medium containing 10% FBS (containing 0.1% DNase) at 37 °C. The mixture was centrifuged at 300 g for 8 min at 25 °C, and the supernatant was removed. The cells were resuspended in a 1640 medium containing 10% FBS (containing 0.1% DNase) at 37 °C in a T75 culture flask and the mixture was left to stand in an incubator at 37 °C and 5% CO₂ for 3 h. Human CD8+ T cells were isolated using the EasySep^{™} human CD8+ T cell enrichment kit and the operation was carried out as per the instructions. The density of human CD8+ T cells was adjusted to 5 × 10⁵ cells/mL using a 1640 medium containing 10% FBS, and the cells were used as effector cells. Tumor cells were resuspended in a 1640 medium containing 10% FBS, and the density was adjusted to 1 × 10⁵ cells/mL. The tumor cell suspension and the human CD8+ T cell suspension were well mixed at a ratio of 1:1, and the resulting cell suspension and a test antibody diluted in gradient were added to a (public) 96-well round-bottom culture plate (with a cover) at 200 µL/well. The cells were placed in a carbon dioxide incubator and stimulated at 37 °C for 72 h. The cells were centrifuged at 400 g for 5 min, the supernatant was removed, and PBS (containing 1:100 diluted PE anti-human CD8) was added at 100 µL/well to resuspend the cells. The mixture was left to stand at 4 °C for 30 min and washed, and the values were read using a flow cytometer (BD, FACS CELESTA).

### Results

In experiments performed using the assay described above, exemplary antibodies could effectively stimulate CD8+ T cell proliferation *in vitro* in the presence of CD79b-positive cells (see FIGs. 14A, 14B, and 14C); whereas the exemplary antibodies did not show CD79b-independent and non-specific proliferation of CD8+ T cells in the presence of CD79b-negative NUGC4 cells (see FIG. 14D).

### Example 16. Assays on Promotion of Proliferation Capacity of Human CD4+ T Cells by Exemplary Antibodies

Exemplary antibodies could dose-dependently stimulate human CD4+ T cell proliferation *in vitro* in the presence of CD79b-positive tumor cells; whereas the exemplary antibodies did not show CD79b-independent and non-specific proliferation of CD4+ T cells in the presence of CD79b-negative NUGC4 cells.

### Experimental procedures

Isolation of human CD4+ T cells: PBMCs were taken out from a liquid nitrogen tank, rapidly thawed at 37 °C, and added dropwise to 10 mL of 1640 medium containing 10% FBS (containing 0.1% DNase) at 37 °C. The mixture was centrifuged at 300 g for 8 min at 25 °C, and the supernatant was removed. The cells were resuspended in a 1640 medium containing 10% FBS (containing 0.1% DNase) at 37 °C in a T75 culture flask and the mixture was left to stand in an incubator at 37 °C and 5% CO₂ for 3 h. Human CD4+ T cells were isolated using the EasySep^{™} human CD4+ T cell enrichment kit and the operation was carried out as per the instructions. The density of human CD4+ T cells was adjusted to 5 × 10⁵ cells/mL using a 1640 medium containing 10% FBS, and the cells were used as effector cells. Tumor cells were resuspended in a 1640 medium containing 10% FBS, and the density was adjusted to 1 × 10⁵ cells/mL. The tumor cell suspension and the human CD4+ T cell suspension were well mixed at a ratio of 1:1, and the resulting cell suspension and a test antibody diluted in gradient were added to a (public) 96-well round-bottom culture plate (with a cover) at 200 µL/well. The cells were placed in a carbon dioxide incubator and stimulated at 37 °C for 72 h. The cells were centrifuged at 400 g for 5 min, the supernatant was removed, and PBS (containing 1:100 diluted FITC anti-human CD4) was added at 100 µL/well to resuspend the cells. The mixture was left to stand at 4 °C for 30 min and washed, and the values were read using a flow cytometer (BD, FACS CELESTA).

### Results

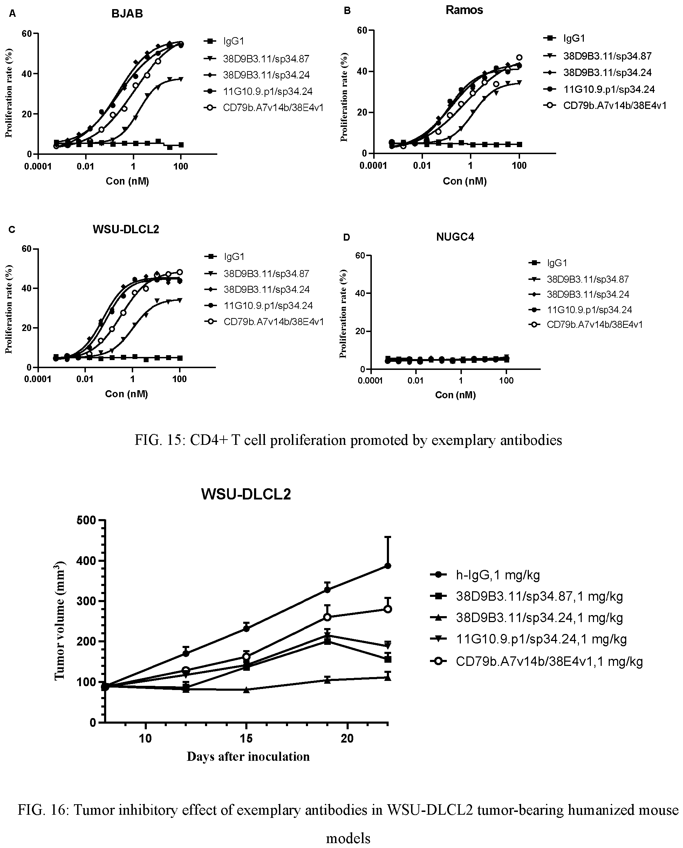

In experiments performed using the assay described above, exemplary antibodies could effectively stimulate CD4+ T cell proliferation *in vitro* in the presence of CD79b-positive cells (see FIGs. 15A, 15B, and 15C); whereas the exemplary antibodies did not show CD79b-independent and non-specific proliferation of CD4+ T cells in the presence of CD79b-negative NUGC4 cells (see FIG. 15D).

### Example 17. Efficacy Assay of Anti-CD79b/CD3 Antibodies of the Present Invention in Animals

In this study, PBMC models of NOG mice were inoculated with the human B-cell non-Hodgkin's lymphoma cell line WSU-DLCl2 and Ramos cells to determine the anti-tumor effect of the exemplary antibodies.

### Anti-tumor effect of exemplary antibodies in WSU-DLCL2 tumor-bearing humanized mouse models

### Experimental procedures

Female NOG mice (14-17 g) were purchased from Beijing Vital River Laboratory Animal Technology Co., Ltd. and were in an SPF grade. The study started after the mice were acclimated and quarantined for 7 days upon arrival.

The mice were intravenously injected with PBMC cells at 4 × 10⁶ cells/mouse in a volume of 200 µL/mouse. WSU-DLCL2 cells were inoculated on the third day after PBMC injection. WSU-DLCL2 cells were passaged conventionally and harvested by centrifugation, and WSU-DLCL2 cells were dispersed in PBS. NOG mice were subjected to shaving at the back and abdomen on the right side and inoculated with WSU-DLCL2 cells at 6 × 10⁶ cells/mouse in a volume of 200 µL/cell.

Administration: On day 8 after inoculation with WSU-DLCL2 cells, the mice were grouped (8 mice per group) and subjected to drug administration according to the tumor volume of the mice. Administration was performed once every 3-4 days for a total of 4 times. The tumor volume and body weight of the mice were monitored twice a week. On day 22 after inoculation, the relative tumor growth inhibition (TGI%) was calculated by the following formula: TGI% = 100 % * (hIgG control group tumor volume - treatment group tumor volume)/(hIgG control group tumor volume - hIgG control group initial tumor volume), wherein the control group initial tumor volume was about 90 mm³. Tumor volume measurement: the maximum length of major axis (L) and maximum length of minor axis (W) of tumors were measured with a vernier caliper, and tumor volume was calculated using the following formula: V = L × W²/2. The mice were weighted using an electronic balance. Throughout the study, the mice were euthanized when the tumors reached an endpoint or when the mice lost more than 20% of body weight. Tumor size was obtained and tumor growth inhibition (TGI%) was calculated.

### Results

Tumor growth curves are shown in FIG. 16, and the exemplary antibodies could significantly inhibit the growth of WSU-DLCL2 cells. The tumor size was obtained on day 22, and tumor growth inhibition was calculated. Compared with hIgG, exemplary antibodies 38D9B3.11/sp34.87, 38D9B3.11/sp34.24, and 11G10.9.p1/sp3424 exhibited 78%, 93%, and 67% tumor growth inhibition, respectively. The TGI of CD79b.A7v14b/38E4v1 was only 36%. Moreover, no significant body weight loss was found in the administered mouse groups.

### Anti-tumor effect of exemplary antibodies in Ramos tumor-bearing humanized mouse models

### Experimental procedures

Female NOG mice (14-17 g) were purchased from Beijing Vital River Laboratory Animal Technology Co., Ltd. and were in an SPF grade. The study started after the mice were acclimated and quarantined for 7 days upon arrival.

PBMC cells were thawed in an RPMI-1640 medium pre-warmed with 0.1% DNase and then dispersed in PBS to prepare a cell suspension with a cell concentration of 20 × 10⁶ cells/mL. Mice were injected intravenously with the PBMC cell suspension at 0.2 mL/mouse, i.e. at an inoculum size of 4 × 10⁶ cells/mouse.

Ramos cells were passaged conventionally for subsequent *in vivo* experiment. Seven days later after PBMC cell inoculation, Ramos cells were dispersed in PBS and matrigel (1:1) to prepare a cell suspension with a cell concentration of 7.5 × 10⁶ cells/mL. The NOG mice were subjected to shaving at the right back and injected subcutaneously with the Ramos cell suspension at 0.2 mL/mouse, i.e., at an inoculum size of 1.5 × 10⁶ cells/mouse.

Six days later after tumor cell inoculation, the mice were grouped (6 mice per group) and subjected to drug administration according to the tumor volume of the mice, and the administration was performed once every 3-4 days for 4 times consecutively. The administration mode was intraperitoneal injection, and the administration volume was 10 mL/kg/time. The tumor volume and body weight of the mice were monitored twice a week, and the monitoring ended on day 20.

On day 20 after inoculation, the relative tumor growth inhibition (TGI%) was calculated by the following formula: TGI% = 100% × (hIgG control group tumor volume - treatment group tumor volume)/(hIgG control group tumor volume - hIgG control group tumor volume before administration). Tumor volume measurement: the maximum length of major axis (L) and maximum length of minor axis (W) of tumors were measured with a vernier caliper, and tumor volume was calculated using the following formula: V = L × W2/2. The mice were weighted using an electronic balance.

### Results

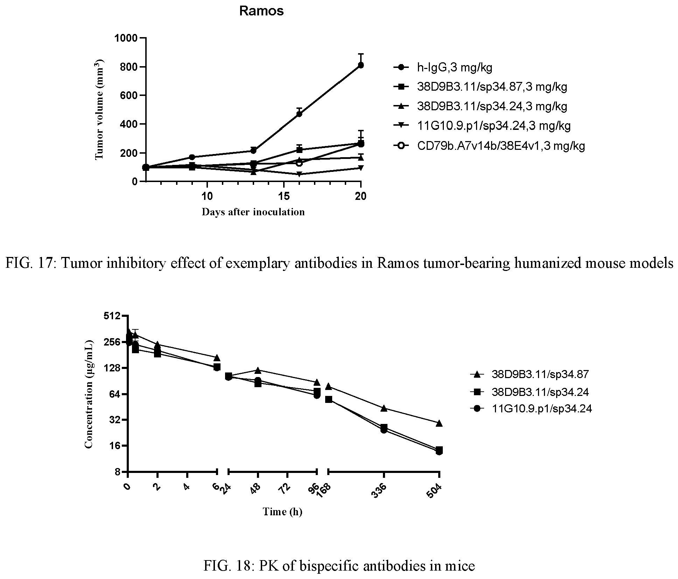

Tumor growth curves are shown in FIG. 17, and the exemplary antibodies could significantly inhibit the growth of Ramos cells. The tumor size was obtained on day 20, and tumor growth inhibition was calculated. Exemplary antibodies 38D9B3.11/sp34.87, 38D9B3.11/sp34.24, and HG10.9.p1/sp34.24 exhibited 76%, 90%, and 100% tumor growth inhibition, respectively. The TGI of CD79b.A7v14b/38E4v1 was 77%, which was similar to that of 38D9B3.11/sp34.87. Moreover, no significant body weight loss was found in the administered mouse groups.

### Example 18. PK Assay of Anti-CD79b/CD3 Antibodies of the Present Invention in Animals

In this study, 38D9B3.11/sp34.87, 38D9B3.11/sp34.24, and 11G10.9.p1/sp3424 were administered to female Balb/C mice at 10 mg/kg by tail vein injection to study their pharmacokinetic properties in the female Balb/C mice. Blood was taken from the eyeball at time points of 0.083 h, 0.5 h, 2 h, 6 h, 24 h, 48 h, 4 days, 7 days, 14 days, and 21 days and centrifuged at 3000 rpm at 4 °C for 10 min, and serum was collected. The half-lives of 38D9B3.11/sp34.87, 38D9B3.11/sp34.24, and 11G10.9.p1/sp3424 in mice was calculated by determining the content of the antibodies in the serum by ELISA. The results are shown in FIG. 18. The half-lives of 38D9B3.11/sp34.87, 38D9B3.11/sp3424, and 11G10.9.p1/sp34.24 in mice were 10.4 days, 7.3 days, and 7.0 days, respectively, and all the three had a PK similar to that of a normal monoclonal antibody.

## Claims

1. An antibody or an antigen-binding fragment thereof that binds to CD79b, comprising a heavy chain variable region VH and a light chain variable region VL, wherein
(i) the VH comprises three complementarity determining regions (CDRs) HCDR1, HCDR2, and HCDR3 contained in a VH set forth in SEQ ID NO: 4, and the VL comprises LCDR1, LCDR2, and LCDR3 contained in a VL set forth in SEQ ID NO: 9;
or
(ii) the VH comprises three complementarity determining regions (CDRs) HCDR1, HCDR2, and HCDR3 contained in a VH set forth in SEQ ID NO: 14, and the VL comprises LCDR1, LCDR2, and LCDR3 contained in a VL set forth in SEQ ID NO: 19.

2. An antibody or an antigen-binding fragment thereof that binds to CD79b, comprising a heavy chain variable region VH and/or a light chain variable region VL, wherein
(i) the VH comprises complementarity determining regions (CDRs) HCDR1, HCDR2, and HCDR3, wherein the HCDR1 comprises or consists of an amino acid sequence set forth in SEQ ID NO: 1 or SEQ ID NO: 11; the HCDR2 comprises or consists of an amino acid sequence set forth in SEQ ID NO: 2 or SEQ ID NO: 12; the HCDR3 comprises or consists of an amino acid sequence set forth in SEQ ID NO: 3 or SEQ ID NO: 13;
and/or
(ii) the VL comprises complementarity determining regions (CDRs) LCDR1, LCDR2, and LCDR3, wherein the LCDR1 comprises or consists of an amino acid sequence set forth in SEQ ID NO: 6 or SEQ ID NO: 16; the LCDR2 comprises or consists of an amino acid sequence set forth in SEQ ID NO: 7 or SEQ ID NO: 17; the LCDR3 comprises or consists of an amino acid sequence set forth in SEQ ID NO: 8 or SEQ ID NO: 18.

3. The antibody or the antigen-binding fragment thereof according to claim 2, comprising a heavy chain variable region VH and a light chain variable region VL, wherein
1) the VH comprises complementarity determining regions (CDRs) HCDR1, HCDR2, and HCDR3, wherein the HCDR1 comprises or consists of the amino acid sequence set forth in SEQ ID NO: 1, the HCDR2 comprises or consists of the amino acid sequence set forth in SEQ ID NO: 2, and the HCDR3 comprises or consists of the amino acid sequence set forth in SEQ ID NO: 3; the VL comprises complementarity determining regions (CDRs) LCDR1, LCDR2, and LCDR3, wherein the LCDR1 comprises or consists of the amino acid sequence set forth in SEQ ID NO: 6, the LCDR2 comprises or consists of the amino acid sequence set forth in SEQ ID NO: 7, and the LCDR3 comprises or consists of the amino acid sequence set forth in SEQ ID NO: 8;
or
2) the VH comprises complementarity determining regions (CDRs) HCDR1, HCDR2, and HCDR3, wherein the HCDR1 comprises or consists of the amino acid sequence set forth in SEQ ID NO: 11, the HCDR2 comprises or consists of the amino acid sequence set forth in SEQ ID NO: 12, and the HCDR3 comprises or consists of the amino acid sequence set forth in SEQ ID NO: 13; the VL comprises complementarity determining regions (CDRs) LCDR1, LCDR2, and LCDR3, wherein the LCDR1 comprises or consists of the amino acid sequence set forth in SEQ ID NO: 16, the LCDR2 comprises or consists of the amino acid sequence set forth in SEQ ID NO: 17, and the LCDR3 comprises or consists of the amino acid sequence set forth in SEQ ID NO: 18.

4. The antibody or the antigen-binding fragment thereof according to any one of claims 1 to 3, comprising a heavy chain variable region VH and/or a light chain variable region VL, wherein
(a) the heavy chain variable region VH
(i) comprises or consists of an amino acid sequence having at least 90%, 91%, 92%, 93%, 94%, 95%, 96%, 97%, 98%, or 99% identity to the amino acid sequence set forth in SEQ ID NO: 4 or SEQ ID NO: 14; or
(ii) comprises or consists of the amino acid sequence set forth in SEQ ID NO: 4 or SEQ ID NO: 14; or
(iii) comprises or consists of an amino acid sequence having 1-10 amino acid replacements, insertions, or deletions compared with the amino acid sequence set forth in SEQ ID NO: 4 or SEQ ID NO: 14;
and/or
(b) the light chain variable region VL
(i) comprises or consists of an amino acid sequence having at least 90%, 91%, 92%, 93%, 94%, 95%, 96%, 97%, 98%, or 99% identity to the amino acid sequence set forth in SEQ ID NO: 9 or SEQ ID NO: 19;
(ii) comprises or consists of the amino acid sequence set forth in SEQ ID NO: 9 or SEQ ID NO: 19; or
(iii) comprises or consists of an amino acid sequence having 1-10 amino acid replacements, insertions, or deletions compared with the amino acid sequence set forth in SEQ ID NO: 9 or SEQ ID NO: 19.

5. The antibody or the antigen-binding fragment thereof according to claim 4, comprising
(1) a heavy chain variable region VH comprising or consisting of an amino acid sequence having at least 90%, 91%, 92%, 93%, 94%, 95%, 96%, 97%, 98%, or 99% identity to the amino acid sequence set forth in SEQ ID NO: 4, and a light chain variable region VL comprising or consisting of an amino acid sequence having at least 90%, 91%, 92%, 93%, 94%, 95%, 96%, 97%, 98%, or 99% identity to the amino acid sequence set forth in SEQ ID NO: 9; or
(2) a heavy chain variable region VH comprising or consisting of an amino acid sequence having at least 90%, 91%, 92%, 93%, 94%, 95%, 96%, 97%, 98%, or 99% identity to the amino acid sequence set forth in SEQ ID NO: 14, and a light chain variable region VL comprising or consisting of an amino acid sequence having at least 90%, 91%, 92%, 93%, 94%, 95%, 96%, 97%, 98%, or 99% identity to the amino acid sequence set forth in SEQ ID NO: 19.

6. The antibody or the antigen-binding fragment thereof according to claim 5, comprising
(1) a heavy chain variable region VH comprising or consisting of the amino acid sequence set forth in SEQ ID NO: 4 and a light chain variable region VL comprising or consisting of the amino acid sequence set forth in SEQ ID NO: 9;
or
(2) a heavy chain variable region VH comprising or consisting of the amino acid sequence set forth in SEQ ID NO: 14 and a light chain variable region VL comprising or consisting of the amino acid sequence set forth in SEQ ID NO: 19.

7. The antibody or the antigen-binding fragment thereof according to any one of claims 1 to 6, comprising a heavy chain and/or a light chain, wherein
(a) the heavy chain
(i) comprises or consists of an amino acid sequence having at least 85%, 90%, 91%, 92%, 93%, 94%, 95%, 96%, 97%, 98%, or 99% identity to an amino acid sequence set forth in SEQ ID NO: 5 or SEQ ID NO: 15;
(ii) comprises or consists of the amino acid sequence set forth in SEQ ID NO: 5 or SEQ ID NO: 15; or
(iii) comprises or consists of an amino acid sequence having 1-10 amino acid replacements, insertions, or deletions compared with the amino acid sequence set forth in SEQ ID NO: 5 or SEQ ID NO: 15;
and/or
(b) the light chain
(i) comprises or consists of an amino acid sequence having at least 85%, 90%, 91%, 92%, 93%, 94%, 95%, 96%, 97%, 98%, or 99% identity to an amino acid sequence set forth in SEQ ID NO: 10 or SEQ ID NO: 20;
(ii) comprises or consists of the amino acid sequence set forth in SEQ ID NO: 10 or SEQ ID NO: 20; or
(iii) comprises or consists of an amino acid sequence having 1-10 amino acid replacements, insertions, or deletions compared with the amino acid sequence set forth in SEQ ID NO: 10 or SEQ ID NO: 20.

8. The antibody or the antigen-binding fragment thereof according to claim 7, comprising
(1) a heavy chain comprising or consisting of an amino acid sequence having at least 85%, 90%, 91%, 92%, 93%, 94%, 95%, 96%, 97%, 98%, or 99% identity to the amino acid sequence set forth in SEQ ID NO: 5, and a light chain comprising or consisting of an amino acid sequence having at least 85%, 90%, 91%, 92%, 93%, 94%, 95%, 96%, 97%, 98%, or 99% identity to the amino acid sequence set forth in SEQ ID NO: 10;
or
(2) a heavy chain comprising or consisting of an amino acid sequence having at least 85%, 90%, 91%, 92%, 93%, 94%, 95%, 96%, 97%, 98%, or 99% identity to the amino acid sequence set forth in SEQ ID NO: 15, and a light chain comprising or consisting of an amino acid sequence having at least 85%, 90%, 91%, 92%, 93%, 94%, 95%, 96%, 97%, 98%, or 99% identity to the amino acid sequence set forth in SEQ ID NO: 20.

9. The antibody or the antigen-binding fragment thereof according to claim 8, comprising
(1) a heavy chain comprising or consisting of the amino acid sequence set forth in SEQ ID NO: 5 and a light chain comprising or consisting of the amino acid sequence set forth in SEQ ID NO: 10;
or
(2) a heavy chain comprising or consisting of the amino acid sequence set forth in SEQ ID NO: 15 and a light chain comprising or consisting of the amino acid sequence set forth in SEQ ID NO: 20.

10. The antibody or the antigen-binding fragment thereof according to any one of claims 1 to 9, wherein the antibody is an antibody in the form of IgG1.

11. The antibody or the antigen-binding fragment thereof according to any one of claims 1 to 10, wherein the antibody is a monoclonal antibody molecule, a chimeric antibody molecule, a humanized antibody or human antibody molecule, or a bispecific antibody or multispecific antibody molecule, and the antigen-binding fragment is one of: a Fab, a Fab', a Fab'-SH, an Fv, a single-chain antibody (e.g., scFv) or (Fab')₂, a single-domain antibody, a diabody (dAb), a camelid antibody (a heavy chain antibody), or a linear antibody.

12. The antibody or the antigen-binding fragment thereof according to claims 1-11, being a bispecific antibody comprising a first heavy chain, a first light chain, a second heavy chain, and a second light chain, wherein the first heavy chain and the first light chain comprise an antigenic site binding to CD79b, and preferably the second heavy chain and the second light chain comprise an antigen-binding site binding to CD3.

13. The antibody or the antigen-binding fragment thereof according to claim 12, wherein
1) the first light chain comprises LCDR1, LCDR2, and LCDR3, wherein the LCDR1 comprises or consists of an amino acid sequence of SEQ ID NO: 6, the LCDR2 comprises or consists of an amino acid sequence of SEQ ID NO: 7, and the LCDR3 comprises or consists of an amino acid sequence of SEQ ID NO: 8; the first heavy chain comprises HCDR1, HCDR2, and HCDR3, wherein the HCDR1 comprises or consists of an amino acid sequence of SEQ ID NO: 1, the HCDR2 comprises or consists of an amino acid sequence of SEQ ID NO: 2, and the HCDR3 comprises or consists of an amino acid sequence of SEQ ID NO: 3; the second heavy chain comprises HCDR1, HCDR2, and HCDR3, wherein the HCDR1 comprises or consists of an amino acid sequence of SEQ ID NO: 31, the HCDR2 comprises or consists of an amino acid sequence of SEQ ID NO: 32, and the HCDR3 comprises or consists of an amino acid sequence of SEQ ID NO: 33; the second light chain comprises LCDR1, LCDR2, and LCDR3, wherein the LCDR1 comprises or consists of an amino acid sequence of SEQ ID NO: 36, the LCDR2 comprises or consists of an amino acid sequence of SEQ ID NO: 37, and the LCDR3 comprises or consists of an amino acid sequence of SEQ ID NO: 38;
2) the first light chain comprises LCDR1, LCDR2, and LCDR3, wherein the LCDR1 comprises or consists of an amino acid sequence of SEQ ID NO: 6, the LCDR2 comprises or consists of an amino acid sequence of SEQ ID NO: 7, and the LCDR3 comprises or consists of an amino acid sequence of SEQ ID NO: 8; the first heavy chain comprises HCDR1, HCDR2, and HCDR3, wherein the HCDR1 comprises or consists of an amino acid sequence of SEQ ID NO: 1, the HCDR2 comprises or consists of an amino acid sequence of SEQ ID NO: 2, and the HCDR3 comprises or consists of an amino acid sequence of SEQ ID NO: 3; the second heavy chain comprises HCDR1, HCDR2, and HCDR3, wherein the HCDR1 comprises or consists of an amino acid sequence of SEQ ID NO: 41, the HCDR2 comprises or consists of an amino acid sequence of SEQ ID NO: 42, and the HCDR3 comprises or consists of an amino acid sequence of SEQ ID NO: 43; the second light chain comprises LCDR1, LCDR2, and LCDR3, wherein the LCDR1 comprises or consists of an amino acid sequence of SEQ ID NO: 46, the LCDR2 comprises or consists of an amino acid sequence of SEQ ID NO: 47, and the LCDR3 comprises or consists of an amino acid sequence of SEQ ID NO: 48;
3) the first light chain comprises LCDR1, LCDR2, and LCDR3, wherein the LCDR1 comprises or consists of an amino acid sequence of SEQ ID NO: 16, the LCDR2 comprises or consists of an amino acid sequence of SEQ ID NO: 17, and the LCDR3 comprises or consists of an amino acid sequence of SEQ ID NO: 18; the first heavy chain comprises HCDR1, HCDR2, and HCDR3, wherein the HCDR1 comprises or consists of an amino acid sequence of SEQ ID NO: 11, the HCDR2 comprises or consists of an amino acid sequence of SEQ ID NO: 12, and the HCDR3 comprises or consists of an amino acid sequence of SEQ ID NO: 13; the second heavy chain comprises HCDR1, HCDR2, and HCDR3, wherein the HCDR1 comprises or consists of an amino acid sequence of SEQ ID NO: 31, the HCDR2 comprises or consists of an amino acid sequence of SEQ ID NO: 32, and the HCDR3 comprises or consists of an amino acid sequence of SEQ ID NO: 33; the second light chain comprises LCDR1, LCDR2, and LCDR3, wherein the LCDR1 comprises or consists of an amino acid sequence of SEQ ID NO: 36, the LCDR2 comprises or consists of an amino acid sequence of SEQ ID NO: 37, and the LCDR3 comprises or consists of an amino acid sequence of SEQ ID NO: 38;
4) the first light chain comprises LCDR1, LCDR2, and LCDR3, wherein the LCDR1 comprises or consists of an amino acid sequence of SEQ ID NO: 16, the LCDR2 comprises or consists of an amino acid sequence of SEQ ID NO: 17, and the LCDR3 comprises or consists of an amino acid sequence of SEQ ID NO: 18; the first heavy chain comprises HCDR1, HCDR2, and HCDR3, wherein the HCDR1 comprises or consists of an amino acid sequence of SEQ ID NO: 11, the HCDR2 comprises or consists of an amino acid sequence of SEQ ID NO: 12, and the HCDR3 comprises or consists of an amino acid sequence of SEQ ID NO: 13; the second heavy chain comprises HCDR1, HCDR2, and HCDR3, wherein the HCDR1 comprises or consists of an amino acid sequence of SEQ ID NO: 41, the HCDR2 comprises or consists of an amino acid sequence of SEQ ID NO: 42, and the HCDR3 comprises or consists of an amino acid sequence of SEQ ID NO: 43; the second light chain comprises LCDR1, LCDR2, and LCDR3, wherein the LCDR1 comprises or consists of an amino acid sequence of SEQ ID NO: 46, the LCDR2 comprises or consists of an amino acid sequence of SEQ ID NO: 47, and the LCDR3 comprises or consists of an amino acid sequence of SEQ ID NO: 48;
5) the first light chain comprises LCDR1, LCDR2, and LCDR3, wherein the LCDR1 comprises or consists of an amino acid sequence of SEQ ID NO: 26, the LCDR2 comprises or consists of an amino acid sequence of SEQ ID NO: 27, and the LCDR3 comprises or consists of an amino acid sequence of SEQ ID NO: 28; the first heavy chain comprises HCDR1, HCDR2, and HCDR3, wherein the HCDR1 comprises or consists of an amino acid sequence of SEQ ID NO: 21, the HCDR2 comprises or consists of an amino acid sequence of SEQ ID NO: 22, and the HCDR3 comprises or consists of an amino acid sequence of SEQ ID NO: 23; the second heavy chain comprises HCDR1, HCDR2, and HCDR3, wherein the HCDR1 comprises or consists of an amino acid sequence of SEQ ID NO: 31, the HCDR2 comprises or consists of an amino acid sequence of SEQ ID NO: 32, and the HCDR3 comprises or consists of an amino acid sequence of SEQ ID NO: 33; the second light chain comprises LCDR1, LCDR2, and LCDR3, wherein the LCDR1 comprises or consists of an amino acid sequence of SEQ ID NO: 36, the LCDR2 comprises or consists of an amino acid sequence of SEQ ID NO: 37, and the LCDR3 comprises or consists of an amino acid sequence of SEQ ID NO: 38;
or
6) the first light chain comprises LCDR1, LCDR2, and LCDR3, wherein the LCDR1 comprises or consists of an amino acid sequence of SEQ ID NO: 26, the LCDR2 comprises or consists of an amino acid sequence of SEQ ID NO: 27, and the LCDR3 comprises or consists of an amino acid sequence of SEQ ID NO: 28; the first heavy chain comprises HCDR1, HCDR2, and HCDR3, wherein the HCDR1 comprises or consists of an amino acid sequence of SEQ ID NO: 21, the HCDR2 comprises or consists of an amino acid sequence of SEQ ID NO: 22, and the HCDR3 comprises or consists of an amino acid sequence of SEQ ID NO: 23; the second heavy chain comprises HCDR1, HCDR2, and HCDR3, wherein the HCDR1 comprises or consists of an amino acid sequence of SEQ ID NO: 41, the HCDR2 comprises or consists of an amino acid sequence of SEQ ID NO: 42, and the HCDR3 comprises or consists of an amino acid sequence of SEQ ID NO: 43; the second light chain comprises LCDR1, LCDR2, and LCDR3, wherein the LCDR1 comprises or consists of an amino acid sequence of SEQ ID NO: 46, the LCDR2 comprises or consists of an amino acid sequence of SEQ ID NO: 47, and the LCDR3 comprises or consists of an amino acid sequence of SEQ ID NO: 48.

14. The antibody or the antigen-binding fragment thereof according to claims 12-13, wherein
1) the first light chain comprises a VL set forth in SEQ ID NO: 9, the first heavy chain comprises a VH set forth in SEQ ID NO: 4, the second heavy chain comprises a VH set forth in SEQ ID NO: 34, and the second light chain comprises a VL set forth in SEQ ID NO: 39;
2) the first light chain comprises a VL set forth in SEQ ID NO: 9, the first heavy chain comprises a VH set forth in SEQ ID NO: 4, the second heavy chain comprises a VH set forth in SEQ ID NO: 44, and the second light chain comprises a VL set forth in SEQ ID NO: 49;
3) the first light chain comprises a VL set forth in SEQ ID NO: 19, the first heavy chain comprises a VH set forth in SEQ ID NO: 14, the second heavy chain comprises a VH set forth in SEQ ID NO: 34, and the second light chain comprises a VL set forth in SEQ ID NO: 39;
4) the first light chain comprises a VL set forth in SEQ ID NO: 19, the first heavy chain comprises a VH set forth in SEQ ID NO: 14, the second heavy chain comprises a VH set forth in SEQ ID NO: 44, and the second light chain comprises a VL set forth in SEQ ID NO: 49;
5) the first light chain comprises a VL set forth in SEQ ID NO: 29, the first heavy chain comprises a VH set forth in SEQ ID NO: 24, the second heavy chain comprises a VH set forth in SEQ ID NO: 34, and the second light chain comprises a VL set forth in SEQ ID NO: 39;
or
6) the first light chain comprises a VL set forth in SEQ ID NO: 29, the first heavy chain comprises a VH set forth in SEQ ID NO: 24, the second heavy chain comprises a VH set forth in SEQ ID NO: 44, and the second light chain comprises a VL set forth in SEQ ID NO: 49.

15. The antibody or the antigen-binding fragment thereof according to claims 12-14, wherein
1) the first light chain comprises or consists of the sequence of SEQ ID NO: 10; the first heavy chain comprises or consists of SEQ ID NO: 5; the second heavy chain comprises or consists of SEQ ID NO: 35; the second light chain comprises or consists of SEQ ID NO: 40;
2) the first light chain comprises or consists of the sequence of SEQ ID NO: 10; the first heavy chain comprises or consists of SEQ ID NO: 5; the second heavy chain comprises or consists of SEQ ID NO: 45; the second light chain comprises or consists of SEQ ID NO: 50;
3) the first light chain comprises or consists of the sequence of SEQ ID NO: 20; the first heavy chain comprises or consists of SEQ ID NO: 15; the second heavy chain comprises or consists of SEQ ID NO: 35; the second light chain comprises or consists of SEQ ID NO: 40;
4) the first light chain comprises or consists of the sequence of SEQ ID NO: 20; the first heavy chain comprises or consists of SEQ ID NO: 15; the second heavy chain comprises or consists of SEQ ID NO: 45; the second light chain comprises or consists of SEQ ID NO: 50;
5) the first light chain comprises or consists of the sequence of SEQ ID NO: 30; the first heavy chain comprises or consists of SEQ ID NO: 25; the second heavy chain comprises or consists of SEQ ID NO: 35; the second light chain comprises or consists of SEQ ID NO: 40;
or
6) the first light chain comprises or consists of the sequence of SEQ ID NO: 30; the first heavy chain comprises or consists of SEQ ID NO: 25; the second heavy chain comprises or consists of SEQ ID NO: 45; the second light chain comprises or consists of SEQ ID NO: 50.

16. An isolated nucleic acid, encoding the antibody or the antigen-binding fragment thereof according to any one of claims 1 to 15.

17. A vector, comprising the nucleic acid according to claim 16, wherein preferably, the vector is an expression vector.

18. A host cell, comprising the nucleic acid according to claim 16 or the vector according to claim 17.

19. The host cell according to claim 18, wherein the host cell is prokaryotic or eukaryotic; preferably, the host cell is a yeast cell, a mammalian cell, or other cells suitable for preparing an antibody or an antigen-binding fragment thereof, wherein preferably, the mammalian cell is a 293 cell or a CHO cell.

20. A method for preparing an antibody or an antigen-binding fragment thereof, comprising culturing the host cell according to any one of claims 18-19 under conditions suitable for expressing the nucleic acid according to claim 16, wherein optionally, the method further comprises isolating the antibody or the antigen-binding fragment thereof, and optionally, the method further comprises recovering the antibody or the antigen-binding fragment thereof from the host cell.

21. An immunoconjugate, comprising the antibody or the antigen-binding fragment thereof according to any one of claims 1 to 15 and an additional substance.

22. A pharmaceutical composition, comprising the antibody or the antigen-binding fragment thereof according to any one of claims 1 to 15 or the immunoconjugate according to claim 21, and optionally a pharmaceutical supplementary material, and optionally one or more other therapeutic agents.

23. Use of the antibody or the antigen-binding fragment thereof according to any one of claims 1 to 15, or the immunoconjugate according to claim 21, or the pharmaceutical composition according to claim 22, in preparing a medicament for the prevention and/or treatment of a disease or a disorder related to CD79b and/or CD3, such as a tumor.

24. The use according to any one of claims 21-22, wherein the antibody or the antigen-binding fragment thereof, the immunoconjugate, or the pharmaceutical composition is capable of being administered in combination with one or more therapies.

25. The use according to claim 23, wherein the therapy is a therapeutic modality and/or other therapeutic agents; preferably, the therapeutic modality comprises surgical treatment and/or radiotherapy.

26. A method for preventing and/or treating a disease or a disorder related to CD79b and/or CD3, comprising administering to a subject an effective amount of the antibody or the antigen-binding fragment thereof according to any one of claims 1-15, the immunoconjugate according to claim 21, or the pharmaceutical composition according to claim 22.

27. A method for detecting antigens CD79b and/or CD3 in a sample, comprising:
(a) contacting the sample with the antibody or the antigen-binding fragment thereof according to any one of claims 1-15; and
(b) detecting a complex formed by the antibody or the antigen-binding fragment thereof and the antigens CD79b and/or CD3, wherein optionally, the antibody is detectably labeled.
